# EUROPÄISCHE PATENTSCHRIFT

(11) **EP 1 532 444 B1**
(45) Veröffentlichungstag und Bekanntmachung des Hinweises auf die Patenterteilung: **18.03.2009**
(21) Anmeldenummer: 03792393.5
(22) Anmeldetag: 20.08.2003
(51) Int. Cl.: G01N 33/50

(54) **VERFAHREN ZUM UNTERSUCHEN VON KÖRPERFLÜSSIKEITEN AUF KREBSZELLEN SOWIE ENTSPRECHENDE ANALYSEKITS**
METHOD FOR ANALYZING BODY FLUIDS FOR THE PRESENCE OF CANCER CELLS AND CORRESPONDING ANALYSIS KITS
METHODE D'ANALYSE DE LIQUIDES ORGANIQUES POUR RECHERCHER D'EVENTUELLES CELLULES CANCEREUSES ET KITS D'ANALYSE CORRESPONDANTS

(30) Priorität: 20.08.2002 DE 10238046
(43) Veröffentlichungstag der Anmeldung: 25.05.2005
(73) Patentinhaber: Giesing, Michael, Prof. Dr. med., 49536 Lienen (DE)
(72) Erfinder: GIESING, Michael, 49536 Lienen (DE); SUCHY, Bernhard, 45663 Recklinghausen (DE)
(74) Vertreter: Reitstötter - Kinzebach
(86) Internationale Anmeldenummer: PCT/EP2003/009229
(87) Internationale Veröffentlichungsnummer: WO 2004/019037

(56) Entgegenhaltungen:
- WO-A-96/29430
- SARTO C ET AL: "MODIFIED EXPRESSION OF PLASMA GLUTATHIONE PEROXIDASE AND MANGANESE SUPEROXIDE DISMUTASE IN HUMAN RENAL CELL CARCINOMA" ELECTROPHORESIS, WEINHEIM, DE, Bd. 20, Nr. 17, November 1999 (1999-11), Seiten 3458-3466, XP001176871 ISSN: 0173-0835
- BOER JUDITH M ET AL: "Identification and classification of differentially expressed genes in renal cell carcinoma by expression profiling on a global human 31,500-element cDNA array" GENOME RESEARCH, COLD SPRING HARBOR LABORATORY PRESS, US, Bd. 11, Nr. 11, November 2001 (2001-11), Seiten 1861-1870, XP002215718 ISSN: 1088-9051
- MOERK H ET AL: "INVERSE MRNA EXPRESSION OF THE SELENOCYSTEINE-CONTAINING PROTEINS GI-GPX AND SEP IN COLORECTAL ADENOMAS COMPARED WITH ADJACENT NORMAL MUCOSA" NUTRITION AND CANCER, LONDON, GB, Bd. 37, Nr. 1, 2000, Seiten 108-116, XP008014221 ISSN: 0163-5581
- SARTO C ET AL: "RENAL CELL CARCINOMA AND NORMAL KIDNEY PROTEIN EXPRESSION" ELECTROPHORESIS, WEINHEIM, DE, Bd. 18, Nr. 3/4, 1997, Seiten 599-604, XP008026280 ISSN: 0173-0835
- GLADYSHEV VADIM N ET AL: "Contrasting patterns of regulation of the antioxidant selenoproteins, thioredoxin reductase, and glutathione peroxidase, in cancer cells" BIOCHEMICAL AND BIOPHYSICAL RESEARCH COMMUNICATIONS, Bd. 251, Nr. 2, 20. Oktober 1998 (1998-10-20), Seiten 488-493, XP002272356 ISSN: 0006-291X
- BRAVARD A ET AL: "Modifications of the antioxidant enzymes in relation to chromosome imbalances in human melanoma cell lines" MELANOMA RESEARCH, Bd. 8, Nr. 4, August 1998 (1998-08), Seiten 329-335, XP009026564 ISSN: 0960-8931
- PESKIN A V ET AL: "SUPER OXIDE DIS MUTASE AND GLUTATHIONE PER OXIDASE ACTIVITIES IN TUMORS" FEBS LETTERS, Bd. 78, Nr. 1, 1977, Seiten 41-45, XP002272358 ISSN: 0014-5793
- KAHLOS KATRIINA ET AL: "Manganese superoxide dismutase in healthy human pleural mesothelium and in malignant pleural mesothelioma" AMERICAN JOURNAL OF RESPIRATORY CELL AND MOLECULAR BIOLOGY, Bd. 18, Nr. 4, April 1998 (1998-04), Seiten 570-580, XP002272359 ISSN: 1044-1549

## Beschreibung

Die vorliegende Erfindung betrifft ein Verfahren zum Untersuchen von Körperflüssigkeiten auf Krebszellen, dessen Verwendung und entsprechende Analysekits sowie die sich daraus ergebenden Behandlungsmöglichkeiten von Krebs. Das Verfahren basiert im Wesentlichen auf der Bestimmung der Mangan-Superoxiddismutase-, Thioredoxinreduktase-, und/oder Glutathionperoxidase-Genexpression. Die Anwendung dieses Verfahrens erlaubt insbesondere eine sichere Tumordiagnose und -prognose. Die Verminderung einer erhöhten Expression dieser Gene kann therapeutischen Wert besitzen und für die Behandlung von Krebs genutzt werden.

Insbesondere aerobe Organismen sind zeitlebens oxidativem Stress ausgesetzt. Sowohl endogene als auch exogene Faktoren führen zur permanenten Entstehung freier Radikale, vor allem in Form reaktiver Sauerstoffspezies. Ohne einen entsprechenden antioxidativen Schutz hätten die mit der Reaktion der freien Radikale einhergehenden Schäden an Zellbestandteilen und zellulären Strukturen alsbald den Tod der Zelle zur Folge.

Wenngleich der Organismus in der Lage ist, den größten Teil oxidativer Schäden zu vermeiden, scheint der sehr komplexe und in jeder einzelnen Zelle aus mehreren hundert Komponenten bestehende antioxidative Schutz nicht lückenlos zu sein. Stattdessen ist davon auszugehen, dass mit zunehmendem Lebensalter oxidative Schäden akkumulieren, was Anlaß zu der Annahme gab, dass dies ein wesentlicher, wenn nicht der entscheidende Faktor des Alterungsprozesses sei. Auch die Entstehung von Krebs wird in diesem Zusammenhang diskutiert.

In menschlichen Gewebe gibt es mindestens drei verschiedene Superoxiddismutasen (kurz SOD). Dazu gehören die zytoplasmatischen Cu/Zn-Superoxiddismutasen und die mitochondriale Mangan-Superoxiddismutase (kurz MNSOD). Diese katalysieren die Zersetzung von Superoxidradikalen (O₂⁻), wobei Wasserstoffperoxid (H₂O₂) entsteht, das wiederum von Katalasen und/oder Glutathionperoxidasen zu H₂O und O₂ zersetzt werden kann.

Es konnte gezeigt werden, dass die Entwicklung von kolorektalen Tumoren und deren Lebermetastasen assoziiert ist mit einem signifikanten Anstieg der MNSOD-Expression (Janssen et al. J.Cancer Res Clin. Oncol. 125(6), 327-35, 1999). Das konnte auch bei Lungentumoren (Chung-man HJ, et al. Cancer Research 1; 61(23), 8578-85, 2001) und bei Brustkrebszellen (Zhongkui Li et al., Free Radical & Medicine 30; 260-267, 2001) gezeigt werden. In klinischen Studien wurde beobachtet, dass ein erhöhter MNSOD-Antigenspiegel bei kolorektalen Karzinomen, bei Magentumoren und bei Glioblastomen ein unabhängiger prognostischer Faktor für die verringerte Überlebensrate der untersuchten Patienten ist (Janssen AML et al. Br. J. Cancer, 78(8) 1051-1057, 1998; Janssen AML et al. Clinical Cancer Research Bd. 6., 3183-3192, 2000; Ria F. et al. British Journal of Cancer 84(4) 529-534, 2001). Andererseits zeigten sich epitheliale Zellen von in situ-Brustkarzinomen und benignen Hyperblasien öfter stark positiv für die MNSOD-Expression als neoplastische epitheliale Zellen von invasiven Brustkarzinomen (Soini Y. et al. J Pathol Sep. 195(2), 156-62. 2001).

Die Thioredoxinreduktase (kurz TXNRD) ist ein Schlüsselenzym für die Regulation des intrazellulären Redoxzustandes. Dieses Enzym katalysiert die NADPH-abhängige Reduktion von Thioredoxindisulfid und einer Vielzahl weiterer oxidierter Zellbestandteile (Becker K, et al. Eur. J. Biochem. 267, 6118-6125, 2000). Eine konstitutive Expression von TXNRD konnte bereits in verschiedenen menschlichen Zelltypen, z.B. Leukozyten, nachgewiesen werden. Neueren Studien zufolge wird der TXNRD-Expression eine Beteiligung an der Entwicklung von Tumoren zugeschrieben (Söderberg A. et al. Cancer Research 60, 2281-2289, 2000).

Der Glutathionperoxidase (kurz GPX) kommt eine wichtige Rolle bei dem Schutz vor oxidativem Stress zu. Dieses Enzym katalysiert die Zersetzung von H₂O₂ zu H₂O und O₂. Eine Überexpression von GPX1 kann daher Zellen vor oxidativer Zerstörung schützen und scheint insbesondere dann wichtig zu sein, wenn auch die MNSOD überexprimiert wird, da es sonst zu einer Akkumulation von H₂O₂ kommen kann (Li S., et al. Cancer Research 60, 3927-3939, 2000). In Imexon-resistenten RPM/8226/I-Myelornzellen beobachtete man eine verringerte GPX1-Expression (Dvorakova K. et al. Molecular Cancer Therapeutics 1, 185-195, 2002).

Bereits 1977 beschrieben Peskin et al. (FEBS LETTERS, Bd. 78, Nr. 1, 1977, S. 41-45) eine verringerte enzymatische Aktivität von Superoxiddismutase und Glutathionperoxidaseaktivitäten in bestimmten Tumorformen, die sie im Kontext eines allgemeinen Nachlassens des aeroben Metabolismus vieler Tumorzellen interpretierten. Im Gegensatz hierzu beschrieben Kahlos et al. (AMERICAN JOURNAL OF RESPI-RATORY CELL AND MOLECULAR BIOLOGY, Bd. 18, Nr. 4, April 1998) eine erhöhte Expression und Aktivität von Mangansuperoxiddismutase und Katalase in menschlichen Mesotheliomzellen. Für menschliche. Melanomzelllinien dagegen zeigten Bravard et al. (MELANOMA RESEARCH, Bd. 8, Nr. 4, Aug. 1998, S. 329-335) einen deletionsbedingten Verlust der Superoxiddismutase-2-Aktivität und paralleles Absinken der Glutathionperoxidase- und Glutathionreduktaseaktivitäten. Gladyshev et al. (BIOCHEMICAL AND BIOPHYSICAL RESEARCH COMMUNICATIONS, Bd. 251, Nr. 2, 20. Okt. 1998, S. 488-493) fanden in verschiedenen murinen und menschlichen Tumoren und Tumorzelllinien unterschiedliche Muster der Regulation von Thioredoxinreduktase und Glutathionperoxidase. Moerk et al. (NUTRITION AND CANCER, London, GB, Bd. 37, Nr. 1, 2000, S. 108-116) untersuchten die Expression von selenocysteinhaltigen Proteinen in Kolontumoren und fanden während der Adenom-Karzinom-Progression eine verringerte Expression von Selenoprotein P, einen variablen Anstieg der gastrointestinalen Glutathionperoxidase und keine Veränderungen der Thioredoxinreduktase α und der Plasmaglutationperoxidase. Sarto et al. ("MODI-FIED EXPRESSION OF PLASMA GLUTATHIONE PEROXIDASE AND MANGANESE SUPEROXIDE DISMUTASE IN HUMAN RENAL CELL CARCINOMA" ELEC-TROPHORESIS, Weinheim, DE, Bd. 20, Nr. 17, Nov. 1999, S. 3458-3466 und "RENAL CELL CARCINOMA AND NORMAL KIDNEY PROTEIN EXPRESSION" E-LECTROPHORESIS, Weinheim, DE, Bd. 18, Nr.3/4, 1997, S. 599-604) untersuchten Nierenbiopsien unter Verwendung zweidimensionaler Polyacrylgelelektrophorese und zeigten das Fehlen von Ubichinon-Cytochrom-C-Reduktase und mitochondrialer NADH-Ubichinonoxidoreduktase sowie die erhöhte Expression von Mangansuperoxiddismutase in renalen Tumoren. Boer et al. ("Identification and classification of differentially expressed genes in renal cell carcinoma by expression profiling on a global human 31,500-element cDNA array", GENOME RESEARCH, Cold Spring Harbor Laboratory Press; US, Bd. 11, Nr. 11, Nov. 2001, S. 1861-1870) wandten ein cDNA-Array mit 31500 Elementen auf Biopsien von insgesamt 37 Patienten an und fanden in Nierentumorzellen insgesamt 1.738 differenziell exprimierte Gene. Die Expression von am Sauerstoff- und Radikalmetabolismus beteiligten Genen war in den Tumorzellen insgesamt verringert.

Alle der genannten Ansätze erfordern die Entnahme von mindestens zwei bioptischen Proben pro Patient, nämlich einer Probe normalen und einer Probe krebsverdächtigen Gewebes. Obgleich eine Vielzahl von Einzelphänomenen beschrieben wurde, ist darunter kein durchgängig für eine Vielzahl von Tumoren verwendbarer Marker zu erkennen.

WO 96/29430 offenbart ein Verfahren zur Detektion von Melanom- oder Brustkrebsmetastasen in einer biologischen Probe unter Verwendung einer RT-PCR auf Melanom- oder brustkrebsspezifische Marker, unter denen Tyrosinase, MUC18, P97, MAGE1, MAGE3, βHCG und GalNAc genannt werden. Dieses Verfahren umgeht die oben dargestellte Notwendigkeit für Doppelbiopsien, erkauft die Möglichkeit des Nachweises disseminierender Tumorzellen jedoch mit Begrenzung auf zwei spezielle Tumorformen (Melanom und Brustkrebs).

Durch die Identifizierung und Charakterisierung disseminierter Krebszellen konnten in den letzten Jahren erstaunliche Fortschritte bei der Diagnose, Prognose und Therapie von Krebs erzielt werden. Dieser Ansatz beruht auf der Erkenntniss, dass die disseminierten Krebszellen eine vom Primärtumor unabhängige Tumorentität sind und daher grundsätzlich von Zellen des Primärtumors aufgrund eines anderen Geno- und Phänotyps zu unterscheiden sind. So gelingt es beispielsweise, mit Hilfe von Multiparameteranalysen unabhängig vom Status des Primärtumors Fragestellungen mit prognostischer und therapeutischer Relevanz bei einer Reihe von Brustkrebspatientinnen zu beantworten (Giesing M. et al., The international Journal of Biological Markers Bd. 15 (1), 94-99, 1999).

Aufgabe der vorliegenden Erfindung ist es zum einen, ein weiteres praktikables Verfahren anzugeben, das eine sichere Krebsdiagnose erlaubt. Vorteilhafterweise sollte das Verfahren auch prognostische Fragestellungen zum weiteren Verlauf einer Krebserkrankung beantworten. Eine weitere Aufgabe der vorliegenden Erfindung ist es, Targets für die medikamentöse Behandlung von Krebs anzugeben.

Gegenstand der vorliegenden Erfindung ist ein Verfahren zum Untersuchen biologischer Proben auf Krebszellen, wobei man an wenigstens einer zellhaltigen Fraktion der biologischen Probe die Expression von wenigstens 2 Genen bestimmt, die ausgewählt sind unter
i) Mangan-Superoxiddismutase-Genen;
ii) Thioredoxinreduktase-Genen; und
iii) Glutathionperoxidase-Genen.

Der Begriff Krebszelle steht erfindungsgemäß für eine Zelle, die eine oder mehrere mit Krebs, also Entartung im allgemeinen Sinn, in Zusammenhang stehende Modifikation aufweist. Grundlage dieser Definition ist die Vorstellung, dass es sich bei der Entstehung von Krebs um einen kontinuierlichen Veränderungsprozess handelt. Beispielsweise bedarf es in der Regel mehrerer Veränderungen, insbesondere des genetischen Materials bzw. der Expression des genetischen Materials von Zellen auf dem Weg von einer Normalzelle zu einer Krebs- und insbesondere zu einer Tumorzelle. Der Begriff Krebszelle umfaßt daher auch Vorstufen von Krebs- und insbesondere Tumorzellen mit krebsartigen bzw. tumorösen Modifikationen.

Die erfindungsgemäße Genexpressionsanalyse beinhaltet die Bestimmung der Expression von wenigstens zwei Genen (Parameter). Die Analyse eines einzelnen Parameters gliedert sich im Wesentlichen in drei Verfahrensschritte:
a) Zweckmäßige Bereitstellung des zu bestimmenden Genexpressionsproduktes;
b) Quantifizierung des Genexpressionsproduktes;
c) Auswertung.

Die Verfahrensschritte a), b) und c) werden vorteilhafterweise in der angegebenen Reihenfolge durchgeführt. Die Untersuchung mehrerer Parameter kann in getrennten Verfahren oder, gemäß einer bevorzugten Ausführungsform der vorliegenden Erfindung, zumindest teilweise parallel in einem entsprechend ausgestalteten Verfahren erfolgen, wobei zumindest die Verfahrensschritte a) und b) für wenigstens 2 der erfindungsgemäßen Parameter i), ii) und iii) parallel durchgeführt werden.

Gemäß einer besonderen Ausführungsform des erfindungsgemäßen Verfahrens wird die Expression wenigstens eines MNSOD-Gens in Kombination mit der Expression wenigstens eines weiteren unter Thioredoxinreduktase-Genen und Gluthationperoxidase-Genen ausgewählten Gens bestimmt. Hiervon ist die Kombination von MNSOD-Genen mit TXNRD-Genen bevorzugt.

Gemäß einer weiteren besonderen Ausführungsform des erfindungsgemäßen Verfahrens wird die Expression wenigstens eines MNSOD-Gens, wenigstens eines TXNRD-Gens und wenigstens eines GPX-Gens bestimmt.

### a) Die Bereitstellung des zu bestimmenden Genexpressionsproduktes

Das erfindungsgemäße Verfahren eignet sich zur Untersuchung von Proben beliebigen biologischen Ursprungs. Eine Ausführungsform betrifft Körperproben humanen und tierischen Ursprungs. Proben wie Gewebe, nativ, gefroren, fixiert, mit und ohne Dissektion, Blut und Blutbestandteile bzw. Isolate davon, weitere Körperflüssigkeiten, z.B. Knochenmark, Lymphe, Sputum, Lavagen, Punktate, Ascites, Schleimhautabstriche, Exsudate und Urin, oder Stuhl, und insbesondere zellhaltige Fraktionen davon, können vorteilhaft mit dem erfindungsgemäßen Verfahren untersucht werden. Demnach handelt es sich um ein in vitro Verfahren.

Gemäß einer bevorzugten Ausführungsform der vorliegenden Erfindung werden Körperflüssigkeiten, insbesondere Blut und Blutbestandteile bzw. Isolate davon, und auch Knochenmark untersucht, in denen gegebenenfalls Krebszellen zugegen sind. Körperflüssigkeiten werden insbesondere auf disseminierte Krebszellen untersucht.

Der Begriff "disseminierten Krebszelle" definiert sich insbesondere im Verhältnis zu soliden Tumoren, also vor allem Primärtumoren, Metastasen und Rezidiven. Im Gegensatz zu soliden Tumoren können disseminierte Krebszellen im Körper eines Individuums zirkulieren. Dies geschieht in der Regel über körpereigene Transportorgane, vor allem Körperflüssigkeiten, insbesondere Blut. In der Regel leiten sich disseminierte Krebszellen von einem soliden Tumor dadurch ab, dass sie zunächst Teil eines soliden Tumors, also insbesondere des Tumorgewebes, sind, von dem sie sich in Folge ablösen. Dadurch verlassen disseminierte Krebszellen den durch den soliden Tumor vorgegebenen Körperbereich, insbesondere die vom Tumor befallenen morphologischen Struktureinheiten, beispielsweise das Organ, und gelangen unter anderem an Orte, zu denen ausgehend vom soliden Tumor kein morphologischer Zusammenhang besteht.

Einem besonderen Aspekt zufolge sind disseminierte Krebszellen gekennzeichnet durch ihre relativ geringe Menge in einer Probe bezogen auf gleichermaßen vorhandene Nichtkrebszellen, d.h. sie machen in der Regel einen vergleichsweise geringen Anteil der zellulären Bestandteile der Probe aus. Man bezeichnet sie daher auch als Restkrebszellen (minimal residual disease, kurz MRD). Betrachtet man beispielsweise zellhaltige Körperflüssigkeiten, so liegt der Anteil disseminierter Krebszellen in der Regel unterhalb von 1:1000, meist unterhalb von 1:10.000 und in vielen Fällen sogar unterhalb von 1:100.000, bezogen auf die Anzahl von Nichtkrebszellen einer zufällig gewonnenen Probe der Körperflüssigkeit. Im Falle von Blut gelten diese Verhältnisse insbesondere in bezug auf mononukleäre Zellen (kurz: MNC).

In der Regel untersucht man disseminierte Krebszellen an zellhaltigen Gemischen, die gegebenenfalls neben Nichtkrebszellen disseminierte Krebszellen aufweisen. Für die erfindungsgemäß durchzuführende Bestimmung der Genexpression können die Gemische unterschiedliche Anteile an disseminierten Krebszellen aufweisen. Krebszellanteile von wenigstens 50 % sind jedoch zweckmäßig, Anteile von wenigstens 70 % bevorzugt und Anteile von wenigstens 80 % von Vorteil.

Mit Blick auf die erfindungsgemäß durchzuführende Genexpressionsanalyse erfolgt erforderlichenfalls eine vorbereitende Aufarbeitung der in der Probe vorhandenen zellulären Bestandteile und insbesondere der zu bestimmenden Genexpressionsprodukte, wodurch diese im Hinblick auf das erfindungsgemäße Verfahren in einer zweckmäßigen Form bereitgestellt werden. Eine solche Aufarbeitung entspricht in der Regel üblicher Praxis und orientiert sich insbesondere an den Erfordernissen für die protein- bzw. nukleinsäureanalytische Expressionsbestimmung.

Eine darüber hinausgehende Anreicherung von Krebszellen und insbesondere von disseminierten Krebszellen kann ebenfalls in an sich bekannter Weise erfolgen, beispielsweise über bekannte Verfahren zur Isolierung von Krebszellen, wie immunspezifische Adsorptionsverfahren, Mikrodissektionsverfahren, Dichtegradientenverfahren oder Filtrationsverfahren.

Unter Isolierung versteht man im Sinne der vorliegenden Erfindung jegliche Anreicherung eines zu isolierenden Bestandteils aus einem Gemisch, das diesen neben wenigstens einem weiteren Bestandteil enthält. Das Ergebnis der Isolierung kann also durchaus ein weiteres Gemisch sein, das aber im Vergleich zum ursprünglichen Gemisch den zu isolierenden Bestandteil im Verhältnis zu wenigstens einem anderen Bestandteil in höherer Konzentration enthält.

Einem besonderen Aspekt zufolge erfolgt die erfindungsgemäße Aufarbeitung unter Anreicherung von Krebszellen. Dieser Aspekt betrifft vor allem die Untersuchung von Körperflüssigkeiten mit relativ geringen Anteilen disseminierter Krebszellen, insbesondere den vorstehend beschriebenen. Ziel dieser Art von Aufarbeitung ist es, Testzellen bzw. in der Regel ein Testzellgemisch bereitzustellen, die bzw. das dann einen höheren Krebszellanteil als die ursprünglichen Zellen oder das ursprüngliche Zellgemisch aufweisen bzw. aufweist, wenn in den ursprünglichen Zellen oder im ursprünglichen Zellgemisch Krebszellen vorhanden sind. Die ursprünglichen Zellen, das ursprüngliche Zellgemisch oder Teile davon können als Vergleichszellen oder Vergleichszellgemisch dienen, die bzw. das dann eine niedrigeren Krebszellanteil als die Testzellen oder das Testzellgemisch aufweisen bzw. aufweist, wenn im ursprünglichen Zellgemisch Krebszellen vorhanden sind.

Ein besonderes Verfahren zur Anreicherung disseminierter Krebszellen wird in WO 00/06702 beschrieben. Dieses Verfahren ist durch Bezugnahme Teil der vorliegenden Offenbarung. Die mit diesem Verfahren anreicherbaren, disseminierten Krebszellen zeichnen sich durch ihren dedifferenzierten, prämetastatischen Charakter aus. Sie werden deshalb auch als Mikrometastasen bezeichnet. Im Gegensatz zu disseminierten Krebszellen, die insbesondere mit immunspezifischen Adsorptionsverfahren anreicherbar sind, d.h. vor allem epitheliale, relativ kleine Krebszellen (insbesondere mit Durchmessem von etwa 20 µm oder weniger) haben die mit dem in WO 00/06702 beschriebenen Verfahren anreicherbaren Krebszellen eine epitheliale-mesenchymale Transition erfahren: sie sind in der Regel größer (insbesondere mit Durchmessern von mehr als etwa 20 µm) und weisen das organotypische Expressionsmuster und/oder die epitheliale Expressionscharakteristik der insbesondere mit immunspezifischen Adsorptionsverfahren anreicherbaren disseminierten Krebszellen in der Regel nicht mehr auf. Während also bei den epithelialen, disseminierten Krebszellen über das organotypische Expressionsmuster und/oder die epitheliale Expressionscharakteristik noch ein gewisser Zusammenhang zum Primärtumor besteht, sind die mesenchymalen, disseminierten Krebszellen unabhängig vom Primärtumor. Dies macht sie zu disseminierten Krebszellen, die erfindungsgemäß bevorzugt zu untersuchen sind.

Bei dem in WO 00/06702 beschriebenen Verfahren werden eine zellhaltige Körperflüssigkeit oder Teile davon, beispielsweise eine unspezifisch angereicherte Fraktion, durch ein Sieb mit einer Maschen- bzw. Porenweite von etwa 10 bis 200 µm geführt und der auf dem Sieb zurückbleibende Siebrückstand, d.h. die auf dem Sieb zurückgehaltene Zellfraktion, gewonnen. Durch Verwendung von Sieben bestimmter Maschen- bzw. Porengröße, die einen größen- und gestaltabhängigen Trennvorgang ermöglichen, wird im Siebrückstand ein Krebszellanteil von wenigstens 50% erreicht, sofern die zellhaltige Körperflüssigkeit oder Teile davon Krebszellen aufweisen. Als Siebe werden bei den bekannten Verfahren flächige oder poröse Gebilde mit Öffnungen verwendet, die so dimensioniert sind, dass in der zellhaltigen Körperflüssigkeit enthaltene Nichtkrebszellen noch passieren können, während Krebszellen bzw. Krebszellaggregate zurück gehalten werden.

Gemäß einer vorteilhaften, eine einfache Automatisierung und Standardisierung des Verfahrens ermöglichenden sowie gleichzeitig die Reinheit der filtrierten Krebszellfraktion weiter erhöhenden Weiterbildung dieses Verfahrens, kann man einen Flachfilter mit einer Maschen- bzw. Porenweite von etwa 10-200 µm verwenden, der in einem Filtergehäuse angeordnet ist, welches durch eine geeignete strömungstechnische Auslegung ein gleichmäßiges Durchspülen der Filterfläche ermöglicht. Dies ist insbesondere in der DE 100 54 632 beschrieben und durch Bezugnahme Teil der vorliegenden Offenbarung.

So befördert man gemäß einer besonderen Ausführungsform die zellhaltige Körperflüssigkeit oder Teile davon in einen Einlassstutzen eines Filtergehäuses, führt die Körperflüssigkeit seitlich aus dem Einlassstutzen in einen einlassseitigen Fluidraum des Filtergehäuses und verteilt sie über einem in den Filtergehäuse angeordneten Flachfilter mit einer Maschen- bzw. Porenweite von etwa 10-200 µm im Wesentlichen parallel zur Oberfläche des Flachfilters, transportiert die Körperflüssigkeit oder die Teile davon über den Flachfilter und trennt sie in einem auf dem Flachfilter verbleibenden Rückstand und ein Filtrat auf, sammelt das Filtrat in einem auslassseitigen Fluidraum und führt es über einen Auslassstutzen ab und gewinnt anschließend den Rückstand.

Sollen Krebszellen aus Blut isoliert werden, so ist erfindungsgemäß bevorzugt, zunächst Zellen des weißen Blutbildes durch Dichtegradientenzentrifugation abzutrennen. Krebszellen findet man vor allem in der Fraktion, die auch mononukleäre Zellen enthält, so dass diese Fraktion bevorzugt (im Folgenden mit MNC-Fraktion bezeichnet) der anschließenden Filtration oder alternativ einem anderen Verfahren zur Isolation von Krebszellen zugeführt wird.

Die Filtration der zellhaltigen Körperflüssigkeit oder der Fraktion ist beendet, wenn die gesamte zellhaltige Flüssigkeit das Sieb bzw. den Flachfilter passiert hat. Es kann sich ein Waschvorgang anschließen, bei dem weitere Flüssigkeit, vorzugsweise Puffer oder Kulturmedium, durch das Sieb bzw. den Flachfilter geführt wird. Die Waschflüssigkeit kann zu dem zuvor gewonnenen Filtrat gegeben oder auch getrennt davon gesammelt und gegebenenfalls verworfen werden.

Die auf dem Sieb bzw. Flachfilter zurückgehaltene Zellfraktion kann direkt der sich anschließenden Expressionsanalyse oder zunächst der Aufbewahrung zugeführt werden. Vorteilhafterweise wird der die Krebszellen enthaltende Rückstand zunächst von dem Sieb bzw. Flachfilter abgelöst und gesammelt. Je nach Art der anschließenden Verwendung kann man zu diesem Zweck verschiedene Vorgehensweisen wählen.

Man kann beispielsweise den Rückstand in einer Lösung inkubieren, die zur Lyse der Zellen führt und die Gewinnung von Zellbestandteilen, wie Nukleinsäuren, Proteinen oder Lipiden erlaubt. Dabei ist es vorteilhaft, wenn die Lösung während der Inkubation bewegt wird. Bei einer manuellen Ausführung des erfindungsgemäßen Verfahrens kann man beispielsweise jeweils einen Spritzenkolben am Einlass- und am Auslassstutzen der Filtervorrichtung anschließen und die Lösung zwischen den beiden Spritzen hin und her pumpen. Bei einem automatisierten Verfahrensablauf kann eine entsprechende Bewegung der Lösung durch Fördermittel, wie beispielsweise Pumpen realisiert werden.

Zur Gewinnung vitaler Krebszellen löst man den am Flachfilter anhaftenden Rückstand vorteilhaft durch Rückspülen des Filters mit einer Flüssigkeit ab, die man vom auslassseitigen Fluidraum des Filtergehäuses in den einlassseitigen Fluidraum fördert. Vorteilhaft handelt es sich bei der Rückspülflüssigkeit um eine Pufferlösung oder ein Kulturmedium. Die so gewonnen Krebs- oder Tumorzellen können beispielsweise zur Gewinnung von Zellbestandteilen oder von Vakzinen kultiviert werden. Alternativ können Krebszellen mit Zentrifugalkraft oder mittels sogenannter optischer Pinzetten von der Filteroberfläche abgelöst werden.

Geeignete Siebe bzw. Flachfilter weisen in der Regel eine Maschen- bzw. Porenweite von etwa 10-200 µm, vorzugsweise von 15 bis 30 µm, besonders bevorzugt von 17-27 µm und ganz besonders bevorzugt von etwa 20 µm auf. Vorteilhaft ist der Flachfilter als Membranfilter ausgebildet, wobei typische Filtermaterialien, wie Kunststoffnetzwerke oder Gewebe, mikroporöse Membranfilter, Filterfliese oder Kombinationen davon eingesetzt werden können. Geeignete Filtermaterialien und geeignete Herstellungsverfahren für derartige Filter sind insbesondere in der WO 00/06702 beschreiben. Besonders bevorzugt werden Filter aus lösungsmittelbeständigem Material verwendet, die beispielsweise aus Kunststoffen, wie Polyethylen, Polypropylen, Polytetrafluorethylen, hochfluorierten Polymeren, Vinylidenfluorid, Aminoplasten und insbesondere Polyester bestehen können.

Zur Auswahl des für die Isolierung bestimmter Krebszellen jeweils geeigneten Siebes bzw. Filters kann der Fachmann in Vorversuchen mit immer engermaschigen Sieben bzw. Filtern (beispielsweise in der Reihenfolge 200 µm, 115 µm, 74 µm, 51 µm, 38 µm, 30 µm, 27 µm, 20 µm, 17 µm, 15 µm und 10 µm) einzelne Zellfraktionen gewinnen und diese auf deren therapeutische und diagnostische Relevanz untersuchen. Dabei kann es sich auch als vorteilhaft erweisen, Filterkombinationen einzusetzen, d. h. in obigem Beispiel etwa mit einem 115 µm-Filter weniger relevante, größere Aggregate abzufiltrieren und lediglich die auf einem nachgeschalteten 20 µm-Filter gesammelte Krebszellenfraktion zu analysieren.

Die erfindungsgemäße Expressionsanalyse betrifft die Bestimmung von beliebigen Genexpressionsprodukten wie Proteinen oder Nukleinsäuren und hier insbesondere mRNA bzw. die davon ableitbaren Nukleinsäuren wie cDNA. Zur Gewinnung dieser Genexpressionsprodukte, gegebenenfalls im Gemisch mit weiteren Zellbestandteilen kann auf allgemein bekannte Methoden zurückgreifen. Für Nukleinsäuren wird man insbesondere die für den Bereich der Isolierung und Reinigung von Nukleinsäuren bekanntermaßen geeigneten Methoden und Reagenzien, beispielsweise eine Guanidinisothiocyanat und Phenol enthaltenden Lösung, verwenden (vgl. Lottspeich F. und Zorbas H. (Hrsg.) Bioanalytik, Heidelberg; Berlin: Spektrum, Akad. Verl., 1998, insbesondere Kapitel 21). Insbesondere kann man mRNA in Form von Poly A⁺-mRNA mittels oligo-dT-Säulenchromatographie oder entsprechend ausgerüsteter magnetischer Beads isolieren.

### b) Quantifizierung des Genexpressionsproduktes

Die zur Quantifizierung des jeweiligen Genexpressionsproduktes verwendbaren Methoden richten sich in erster Linie nach der Art des Genexpressionsproduktes. So kann man im Prinzip sämtliche bekanntermaßen zur Quantifizierung von Proteinen und Nukleinsäuren geeignete Verfahren aus den Bereichen Proteinanalytik und Nukleinsäureanalytik einsetzen. Aus dem Bereich Proteinanalytik können beispielsweise enzymatische Aktivitätstests, immunologische Techniken, bestimmte spektroskopische Verfahren und die Massenspektrometrie erforderlichenfalls in Kombination mit chromatographischen oder elektrophoretischen Trennmethoden genannt werden. Um einen spezifischen Nachweis der exprimierten Proteine zu gewährleisten, werden vorteilhafterweise immunologische Verfahren zur Anwendung kommen, wie sie beispielsweise in den eingangs geschilderten Arbeiten zur Expression der Gene MNSOD, TXNRD1 und GPX1 beschrieben sind.

Ausgehend von der jeweiligen Aminosäuresequenz ist der Fachmann insbesondere in der Lage, Antikörper herzustellen, die gegen das Protein gerichtet sind. Dazu kann man das gesamte Protein oder Fragmente davon (Polypeptide) als Immunogen verwenden und in an sich bekannter Weise polyklonale und monoklonale Antikörper, und darauf aufbauend mittels rekombinanter Techniken auch humanisierte Antikörper, sowie Fragmenten davon, herstellen.

Diese Antikörper können dann insbesondere in quantitativen Immunoassays und Immunoblot-Techniken, z.B. dem Western-Blotting, Anwendung finden. Geeignet sind sowohl direkte als auch indirekte Assays. Insbesondere sind kompetitive Immunoassays, d.h. das nachzuweisende Protein oder Polypeptid konkurriert als Antigen mit markiertem Antigen um die Antikörperbindung. Bevorzugt sind Sandwich-Immunoassays, d.h. die Bindung spezifischer Antikörper an das Antigen wird mit einem zweiten, meist markierten Antikörper nachgewiesen. Diese Assays können sowohl homogen, d.h. ohne eine Trennung in feste und flüssige Phase, als auch heterogen, d.h. gebundene Markierungen werden von ungebundenen, beispielsweise über festphasengebundene Antikörper, getrennt, ausgelegt sein. Die verschiedenen heterogenen und homogenen Immunoassay-Formate können je nach Markierung und Meßmethode bestimmten Klassen zugeordnet werden, beispielsweise RIAs (Radio-Immunoassays), ELISA (Enzyme Linked ImmunoSorbant Assay), FIA (Fluoreszenz-Immunoassay), LIA (Lumineszenz-Immunoassay), TRFIA (zeitliche aufgelöster FIA), IMAC (Immunaktivierung), EMIT (Enzyme Multiplied Immune Test), TIA (Turbodimetrischer Immunoassay).

Von den massenspektrometrischen Methoden ist insbesondere das sogenannte SELDI-Verfahren zu nennen. Hierbei werden die zu untersuchenden Proteingemische zunächst auf geeigneten Oberflächen, z.B. festen Trägeroberflächen mit Affinität für Proteine, eingefangen, unerwünschte Substanzen erforderlichenfalls von den Oberflächen entfernt, beispielsweise durch Waschen mit geeigneten Flüssigkeiten, und anschließend mittels MALDI-TOF (Laser Desorption/Ionisation Time-Off Flight Mass Analysis) bestimmt.

Ebenso ist der Fachmann in der Lage, Nukleinsäuren, die dieses Protein oder Teile davon codieren, aufzufinden und geeignete Mittel zu deren spezifischen Nachweis zur Verfügung zu stellen.

So sind aus dem Bereich der Nukleinsäureanalytik insbesondere Methoden zu nennen, die auf einer spezifischen Bindung mit der zu bestimmenden Nukleinsäure beruhen. Hierzu gehören vor allem die spezifische Amplifikation der zu bestimmenden Nukleinsäure oder davon ableitbarer Teile, z.B. die Bestimmung von mRNA mittels quantitativer PCR, und/oder deren spezifische Hybridisierung an gegebenenfalls immobilisierte Sonden (insbesondere mit Hilfe von Nukleinsäure-Arrays, auch Biochips genannt), erforderlichenfalls nach vorheriger spezifischer oder unspezifischer Amplifikation.

Der erfindungsgemäße Begriff "Mangan-Superoxiddismutase" (kurz MNSOD) bezeichnet Enzyme, welche die Zersetzung von Superoxidradikalen (O₂⁻) unter Bildung von Wasserstoffperoxid (H₂O₂) katalysieren. Hierzu gehören insbesondere diejenigen Enzyme, die in der Enzymklasse 1.15.1.1 zusammengefasst werden.

Aufgrund der unterschiedlichen phylogenetischen Entwicklung ergibt sich eine gewisse Species-abhängige Heterogenität innerhalb dieser Gruppe von Enzymen. Je nach zu untersuchendem Individuum wird sich die Bestimmung auf die jeweilige in dem betreffenden Organismus zu erwartende MNSOD richten. Gemäß einer besonderen Ausführungsform der vorliegenden Erfindung richtet sich die Bestimmung auf MNSODs humanen Ursprungs.

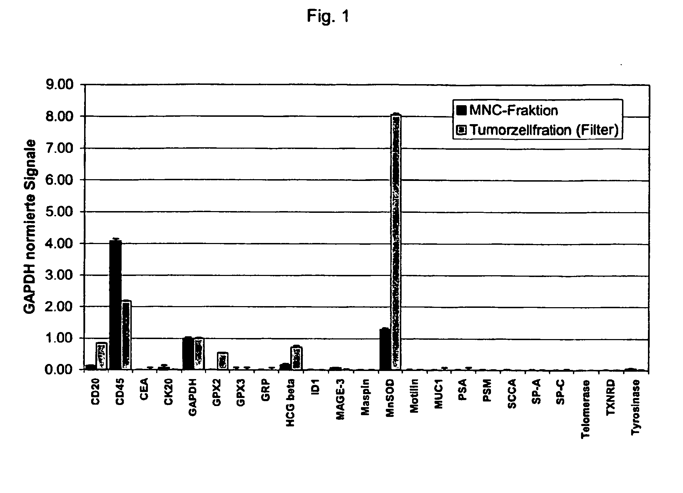

Zusätzlich zu Spezies-abhängigen Variationen finden sich für jede Spezies in der Regel auch polymorphe Varianten, die aufgrund alleler Variation unterschiedliche Aminosäuresequenzen aufweisen. Derartige Variationen wurden bereits beschrieben (Barra et al. (1984) J. Biol. Chem. 259: 12595-12601; US 5,246,847 (Figur 1A); US 5,260,204 (Anspruch 1); US 5,985,633 (SEQ ID NO: 1): der in Stoehlmacher J et al. (2002) Oncol. Rep. 9: 235-238 beschriebene 9Ala-9Val-Polymorphismus). Auf die in diesen Druckschriften beschriebenen MNSODs wird vollumfänglich Bezug genommen.

Gemäß einer besonderen Ausführungsform der vorliegenden Erfindung richtet sich die Expressionsanalyse auf eine MNSOD mit der Aminosäuresequenz SEQ ID NO: 13.

Weitere nützliche Anweisungen zur erfindungsgemäßen Bestimmung von MNSOD kann der Fachmann auch den in den vorstehend genannten Druckschriften angegebenen Nukleinsäuresequenzen entnehmen. Darüber hinaus finden sich zahlreiche Einträge in einschlägigen Gendatenbanken zu MNSOD-codierenden Nukleinsäuresequenzen, von denen ausgehend der Fachmann in der Lage ist, geeignete Mittel zum sequenzspezifischen Nachweis dieser Sequenzen bzw. von davon ableitbaren Expressionsprodukten zur Verfügung zu stellen. Insbesondere sind in diesem Zusammenhang aus menschlichem Lebergewebe isolierbare MNSOD-mRNA (Accession-Nr. X14322), aus humanem Colonkarzinom isolierbare MNSOD-mRNA (Accession-Nm. X59445, X15132, Y00985 und M36693), aus menschlichem Placentagewebe isolierbare MNSOD-mRNA (Accession-Nr. X07834), aus einer humanen T-Zell-DNA-Genbank ableitbare cDNA (Accession-Nr. E01408, vgl. auch JP 1987289187-A1) sowie verschieden MNSOD-Varianten codierende DNAs und RNAs (Accession-Nrn. E03557, E08013 und E08014; vgl. auch JP 1992117288-A1 und JP 1994245763-A1) zu nennen.

Gemäß einer weiteren besonderen Ausführungsform richtet sich der sequenzspezifische Nachweis der MNSOD-Expression auf die Bestimmung einer mRNA bzw. entsprechenden cDNA mit der Sequenz SEQ ID NO:14 oder einer Teilsequenz davon.

Die spezifische Amplifikation dieser Sequenz gelingt beispielsweise mit den Primersequenzen SEQ ID NO:1 und SEQ ID NO:2. Eine geeignete Sonde wird beispielsweise mit SEQ ID NO:3 angegeben. Diese Sonde ist insbesondere für den 5'-Exonuklease-Nachweis unter Verwendung der beiden zuvor genannten Primersequenzen geeignet.

Der erfindungsgemäße Begriff "Thioredoxinreduktase" (kurz TXNRD) bezeichnet Enzyme, welche die NADPH-abhängige Reduktion von Thioredoxin-S₂ zu Thioredoxin-(SH)₂ katalysieren. Hierzu gehören insbesondere diejenigen Enzyme, die in der Enzymklasse 1.6.4.5 zusammengefasst werden.

Hinzu kommt, dass die Thioredoxinreduktase-Familie mehrere Thioredoxinreduktase-Isoformen umfasst, von denen neben der Thioredoxinreduktase 1 insbesondere die Thioredoxinreduktasen des Typs 2 (z.B. α oder β), oder 3 zu nennen sind.

Aufgrund der unterschiedlichen phylogenetischen Entwicklung ergibt sich eine gewisse species-abhängige Heterogenität innerhalb dieser Gruppe von Enzymen. Je nach zu untersuchendem Individuum wird sich die Bestimmung auf die jeweilige in dem betreffenden Organismus zu erwartende TXNRD richten. Gemäß einer besonderen Ausführungsform der vorliegenden Erfindung richtet sich die Bestimmung auf TXNRDs humanen Ursprungs. Zusätzlich zu Spezies-abhängigen Variationen finden sich für jede Spezies in der Regel auch polymorphe Varianten, die aufgrund alleler Variation unterschiedliche Aminosäuresequenzen aufweisen. Auf die in diesen Druckschriften beschriebenen TXNRDs wird vollumfängliche bezug genommen.

Gemäß einer besonderen Ausführungsform der vorliegende Erfindung richtet sich die Expressionsanalyse auf eine TXNRD1 mit der Aminosäuresequenz SEQ ID N0:15.

Weitere nützliche Anweisungen zur erfindungsgemäßen Bestimmung von TXNRDs kann der Fachmann auch den in den vorstehend genannten Druckschriften angegebenen Nukleinsäuresequenzen entnehmen. Darüber hinaus finden sich zahlreiche Einträge in einschlägigen Gendatenbanken zu TXNRD-codierenden Nukleinsäuresequenzen, von denen ausgehend der Fachmann in der Lage ist, geeignete Mittel zum sequenzspezifischen Nachweis dieser Sequenzen bzw. von davon ableitbaren Expressionsprodukten zur Verfügung zu stellen. Insbesondere sind in diesem Zusammenhang TXNRD-mRNA (Accession-Nrn. AF106697, S79851, und AF201385), aus menschlichem Plazentagewebe isolierbare TXNRD-mRNA (Accession-Nrn. X9124), aus menschlichem Himgewebe isolierbare TXNRD-mRNA (Accession-Nr. AF208018), aus menschlichen Osteoblasten isolierbare TXNRD-mRNA (Accession-Nr. AJ001050), aus menschlichem großzelligem Lungenkarzinom isolierbare TXNRD1-mRNA (Accession-Nr. BC018122), aus menschlichem Plazentagewebe isolierbare TXNRD2α-mRNA (Accession-Nr. AB019694) bzw. TXNRD2β-mRNA (Accession-Nr. AB019695), aus humanem Melanom isolierbare TXNRDβ-mRNA (Accession-Nr. BC007489), aus humanem Brustkarzinom isolierbare TXNRD-GRIM-12-mRNA (Accession-Nr. AF077367), TXNRD2-mRNA (Accession-Nr. AF171055) und TXNRD3-mRNA (Accession-Nr. AF133519 und AF171054) zu nennen.

Gemäß einer weiteren besonderen Ausführungsform richtet sich der sequenzspezifische Nachweis der TXNRD-Expression auf die Bestimmung der TXNRD1-Expression und insbesondere einer mRNA bzw. entsprechenden cDNA mit der Sequenz SEQ ID NO:16 oder einer Teilsequenz davon.

Die spezifische Amplifikation dieser Sequenz gelingt beispielsweise mit den Primersequenzen SEQ ID NO:4 und SEQ ID NO:5. Eine geeignete Sonde wird beispielsweise mit SEQ ID NO:6 angegeben. Diese Sonde ist insbesondere für den 5'-Exonuklease-Nachweis unter Verwendung der beiden zuvor genannten Primersequenzen geeignet.

Der erfindungsgemäße Begriff "Glutathionperoxidase" (kurz GPX) bezeichnet Enzyme, welche - ähnlich den Katalasen - die Zersetzung von Wasserstoffperoxid (H₂O₂) unter Bildung von Wasser und Sauerstoff katalysieren. Hierzu gehören insbesondere diejenigen Enzyme, die in der Enzymklasse 1.11.1.9 zusammengefasst werden.

Hinzu kommt, dass die Glutathionperoxidase-Familie mehrere Glutathionperoxidase-Isoformen umfasst, von denen neben der Glutathionperoxidase 1 insbesondere die Glutathionperoxidasen des Typs 2, 3, 4, 5 oder 6 zu nennen sind.

Aufgrund der unterschiedlichen phylogenetischen Entwicklung ergibt sich eine gewisse species-abhängige Heterogenität innerhalb dieser Gruppe von Enzymen. Je nach zu untersuchendem Individuum wird sich die Bestimmung auf die jeweilige in dem betreffenden Organismus zu erwartende GPX richten. Gemäß einer besonderen Ausführungsform der vorliegenden Erfindung richtet sich die Bestimmung auf GPXs humanen Ursprungs.

Zusätzlich zu Spezies-abhängigen Variationen finden sich für jede Spezies in der Regel auch polymorphe Varianten, die aufgrund alleler Variation unterschiedliche Aminosäuresequenzen aufweisen. Derartige Variationen wurden bereits beschrieben; z.B. eine Aminosäuresubstitution Pro-Leu an Position 197 (Forsberg L et al. (1999) Hum. Mutat. 14(4):294-300). Auf die in diesen Druckschriften beschriebenen GPXs wird vollumfänglich Bezug genommen.

gemäß einer besonderen Ausführungsform der vorliegenden Erfindung richtet sich die Expressionsanalyse auf eine GPX1 mit der Aminosäuresequenz SEQ ID NO:17.

Weitere nützliche Anweisungen zur erfindungsgemäßen Bestimmung von GPX kann der Fachmann auch den in den vorstehend genannten Druckschriften angegebenen Nukleinsäuresequenzen entnehmen. Darüber hinaus finden sich zahlreiche Einträge in einschlägigen Gendatenbanken zu GPX-codierenden Nukleinsäuresequenzen, von denen ausgehend der Fachmann in der Lage ist, geeignete Mittel zum sequenzspezifischen Nachweis dieser Sequenzen bzw. von davon ableitbaren Expressionsprodukten zur Verfügung zu stellen. Insbesondere sind in diesem Zusammenhang humane GPX-mRNA (Accession-Nr. AF217787), Exon 1, 2, und 3 bis 5 humane GPX-DNA (Accession-Nrn. D16360, D16361 bzw. D16362), aus menschlichem Plazenta- und fötalem Lebergewebe isolierbare GPX-mRNA (Accession-Nr. D00632), aus menschlichem Lebergewebe isolierbare GPX-mRNA (Accession-Nrn. Y00433 und E02175, vgl. auch JP 1990002362 A1), aus menschlichem Nierengewebe isolierbare GPX-mRNA (Accession-Nr. Y13710, Y00369, X13709 und X13430), aus menschlichem Leukozyten isolierbare GPX-DNA (Accession-Nr. Y00483), aus menschlichem Myelozytleukämie-Zellen isolierbare GPX1-mRNA (Accession-Nr. M21304), aus menschlichem Colonkarzinom isolierbare GPX2-DNA (Accession-Nr. X91863), aus menschlichem Blasenkarzinom isolierbare GPX2-mRNA (Accession-Nr. BC005277 und BC016756), aus menschlichen Fibroblasten isolierbare GPX2-DNA (Accession-Nr. AF199441), aus menschlichem Plazentagewebe isolierbare GPX3-mRNA (Accession-Nr. X58295), aus menschlichem großzelligem Lungenkarzinom isolierbare GPX3-mRNA (Accession-Nr. BC013601), aus menschlichem Milzgewebe isolierbare GPX3-mRNA (Accession-Nr. BC025956), aus menschlichem Hodengewebe isolierbare GPX4-mRNA (Accession-Nr. X71973), aus menschlichem Melanom isolierbare GPX4-mRNA (Accession-Nr. BC010157) und aus menschlichem Epididymisgewebe isolierbare GPX5-mRNA (Accession-Nr. AJ005277) zu nennen.

Gemäß einer weiteren besonderen Ausführungsform richtet sich der sequenzspezifische Nachweis der GPX-Expression auf die Bestimmung der GPX1-Expression und insbesondere einer mRNA bzw. entsprechenden cDNA mit der Sequenz SEQ ID NO: 18 oder einer Teilsequenz davon.

Die spezifische Amplifikation dieser Sequenz gelingt beispielsweise mit den Primersequenzen SEQ ID NO:7 und SEQ ID NO:8. Eine geeignete Sonde wird beispielsweise mit SEQ ID NO:9 angegeben. Diese Sonde ist insbesondere für den 5'-Exonuklease-Nachweis unter Verwendung der beiden zuvor genannten Primersequenzen geeignet.

Der Begriff Amplifikation betrifft die Vervielfältigung von Nukleinsäuren, d.h. die Erzeugung vieler Kopien bestimmter Nukleinsäuren. In der Regel verläuft die Amplifikation wenigstens linear und vorzugsweise exponentiell.

Brauchbar sind die bekannten Amplifikationsverfahren, zu denen die Polymerase-Kettenreaktion (PCR), prinzipiell auch als Nested-PCR, Asymmetrische PCR oder Multiplex-PCR durchgeführt, oder alternative Verfahren, wie die Ligase-Kettenreaktion (LCR), Nukleinsäuresequenz-basierende Amplifikation (NASBA), Transkriptions-vermittelte Amplifikation (TMA) und ähnliche, gehören. Bestimmte Versionen dieser Techniken und/oder Kombinationen mit anderen molelularbiologischen Methoden können zweckmäßig sein.

Vorzugsweise führt man die Amplifikation in Anlehnung an PCR-Techniken durch. Dazu verwendet man in der Regel wenigstens zwei Primer unterschiedlicher Polarität (d.h. wenigstens ein Primerpaar aus einem forward-Primer und einem reverse-Primer) pro Templat.

Gemäß einer besonderen Ausführungsform der vorliegenden Erfindung verwendet man ein Paar spezifischer Primer pro zu bestimmender Nukleinsäuresequenz. Weiterhin ist auch die Möglichkeit gegeben, die gesamte RNA einer Probe zu amplifizieren (vgl. z.B. Zohlnhöfer D. et al. Circulation 103, 1396-1402, 2001) und anschließend bestimmte RNAs als entsprechende cDNAs durch spezifische Hybridisierung zu bestimmen.

Gemäß einer besonderen Ausführungsform der vorliegenden Erfindung verwendet man zur Amplifikation wenigstens einen Primer, der markiert ist. Die Markierung dient zum Nachweis eines Amplifikats, in das der markierte Primer im Zuge der Amplifikation eingebaut wurde.

Dem Fachmann ist eine Vielzahl geeigneter Markierungen samt dazugehöriger Detektionssysteme bekannt. Fluoreszierende und chemi- oder biolumineszierende Markierungen werden aus Gründen der Sensitivität und praktischen Handhabung bevorzugt.

Prinzipiell geeignet sind Markierungssysteme, die sich z.B. spektroskopisch, photochemisch, biochemisch, immunochemisch, elektrisch, optisch oder chemisch erkennen lassen. Dazu gehören sowohl direkte Markierungssysteme, wie radioaktive Marker (z.B. ³²P,³H, ¹²⁵I, ³⁵S, ¹⁴C), magnetische Marker, Chromophore, beispielseise UV-, VIS-, oder IR-absorbierende Verbindungen, Fluorophore, chemi- oder biolumineszierende Marker, Übergangsmetalle, die in der Regel chelatgebunden sind, oder Enzyme, z.B. Meerrettich-Peroxidase oder alkalische Phosphatase und die daran gekoppelten Nachweisreaktionen, als auch indirekte Markierungssysteme, beispielsweise Haptene, wie Biotin oder Digoxigenin, die über entsprechende Nachweissysteme erkannt werden können.

Vorteilhafte Chromophore besitzen eine intensive Farbe, die von den umgebenden Molekülen nur geringfügig absorbiert wird. Farbstoffklassen, wie Chinoline, Triarylmethane, Acridine, Alizarine, Phthaleine, Azoverbindungen, Anthrachinone, Cyanine, Phenazathioniumverbindungen oder Phenazoxoniumverbindungen, seien hier stellvertretend für das breite Spektrum erfindungsgemäß geeigneter Chromophore genannt.

Fluoreszierende Markierungen sind von Vorteil. Man erhält starke Signale mit wenig Hintergrund, hoher Auflösung und hoher Sensitivität. Erfindungsgemäß von Bedeutung ist, daß ein und derselbe Fluorophor je nach Anregung und Detektionsprinzip mehrere unterscheidbare Strahlungen emittieren kann.

Fluorophore können allein oder in Kombination mit einem Quencher (z.B. Molecular Beacons) verwendet werden.

Bevorzugte Fluorophore sind beispielsweise Aminomethylcoumarinessigsäure (AMCA, blau), EDANS, BODIPY 493/503; FL; FL Br2; R6G; 530/550; 558/568; TMR 542/574; TR 589/617; 630/650; 650/665, 6-FAM Fluorescein (grün), 6-OREGON green 488, TET, Cy3 (rot), Rhodamine (rot), 6-JOE, VIC, HEX, 5-TAMRA, NED, 6-ROX, TEXAS Red7 (rot), Cy5, Cy5.5, LaJolla Blue, Cy7, Alexa-Fluor-Carbonsäuren, insbesondere des Typs 647 und 532, z.B. als Succinimidylester, und IRD41.

Besonders bevorzugte Fluorophore sind Cy5, 5-Tamra und Cy3 sowie Alexa-Fluor-Carbonsäuren.

Chemilumineszierende oder biolumineszierende Markierungen sind ebenfalls von Vorteil. Bevorzugte Markierungen dieser Art basieren beispielsweise auf Reaktionen der Alkalischen Phosphatase mit Dioxetan-(AMPPD) oder Acridiniumphosphat-Substraten; der Meerrettichperoxidase mit Luminol- oder Acridiniumester-Substraten; von Mikroperoxidasen oder Metallporphirinsystemen mit Luminol; der Glucoseoxidase, der Glucose-6-phosphatdehydrogenase; oder auch Luciferin/Luciferase-Systemen.

Eine Markierung kann im Prinzip auf beliebige Art und Weise in ein nachzuweisendes Amplifikat eingeführt werden, solange sie den Nachweis desselben erlaubt. Grundsätzlich kann man zwischen einer direkten und indirekten Markierung unterscheiden. Bei der direkten Markierung wird die nachweisbare Markierung selbst im Zuge der Amplifikation eingebaut. Bei der indirekten Markierung wird zunächst ein primäres Label eingebaut, welches eine gewisse Affinität für die anschließend zuzugebende nachweisbare Markierung besitzt. Letztere Vorgehensweise ist immer dann von Vorteil, wenn die zu verwendende Markierung den Verlauf der Amplifikation beeinflussen könnte. Insbesondere im Fall von chemi- oder biolumineszierenden Markierungen ist die indirekte Vorgehensweise bevorzugt. Erfindungsgemäß haben sich vor allem Biotin/Streptavidin-Systeme als zweckmäßig erwiesen. Demnach sind die erfindungsgemäß zu verwendenden markierten Primer gemäß einer besonderen Ausführungsform der vorliegenden Erfindung mit Biotin oder Digoxigenin, vorzugsweise am 5'-Ende, markiert.

Werden erfindungsgemäß mindestens zwei verschiedene Nukleinsäuren bestimmt, so ist es in der Regel von Vorteil, eine sogenannte Multiplex-Amplifikation, also insbesondere Multiplex-PCR, durchzuführen. Dabei sollen mindestens zwei verschiedene Nukleinsäuren der spezifischen Amplifikation unterworfen werden, wobei dies in einem gemeinsamen Ansatz stattfindet. Insbesondere werden bei der Multiplex-PCR mit Hilfe von wenigstens zwei jeweils spezifischen Primerpaaren ebenso viele verschiedene Amplifikate generiert, sofern die entsprechenden Template zugegen sind. Dabei können die Primer bzw. die Primerpaare obigen Ausführungen entsprechend ausgestaltet werden. Insbesondere ist es erfindungsgemäß bevorzugt, sämtliche Primer bzw. Primerpaare in gleicher Weise auszugestalten, so dass die generierten Amplifikate allesamt in den nachfolgenden Verfahrensschritten in analoger Weise behandelt werden können. Insbesondere ist es von Vorteil, die Amplifikate in einem gemeinsam Verfahrensschritt detektieren zu können.

Darüber hinaus kann ein solcher Multiplex-Ansatz weitere Amplifikationssysteme mit einbeziehen, die insbesondere einer Kontrolle dienen können. So kann es insbesondere erwünscht sein, Normalisierungs-, Mismatch-, Housekeeping-, Probenzubereitungs-, Hybridisierungs- oder Amplifikationskontrollen mit einzubeziehen.

Insbesondere ist es erfindungsgemäß bevorzugt, die von den betreffenden Genen transkribierte mRNA zu bestimmen. Dabei kann es sich um bereits gespleißte oder noch nicht gespleißte mRNA handeln. Vorteilhafterweise ist die Bestimmung auf gespleißte mRNA gerichtet.

In der Regel wird nicht die mRNA direkt nachgewiesen, sondern davon ableitbare Nukleinsäuren. Dies gilt insbesondere für den Fall, dass der Nachweis eine Amplifikation mittels PCR beinhaltet. Dazu wird die mRNA üblicherweise zunächst in DNA umgeschrieben. Dies gelingt beispielsweise mittels Reverser Transkription, wobei die zur mRNA komplementäre DNA (cDNA) erzeugt wird. Geeignete Vorgehensweisen zur Erzeugung von cDNA aus mRNA sind geläufiges Fachwissen.

Die resultierende cDNA als Maß für die vorhandene Menge an entsprechender mRNA kann anschließend mittels PCR quantifiziert werden. Auch hier sind dem Fachmann geeignete Methoden bekannt (siehe beispielsweise das Kapitel 24.2.5 in Lottspeich F. und Zorbas H. (Hrsg.) Bioanalytik, Heidelberg; Berlin: Spektrum, Akad. Verl., 1998, insbesondere Kapitel 21). Als besonders effektiv hat sich in diesem Zusammenhang die unter dem Fachbegriff "5'-Exonuklease-Assay" bekannte Technik erwiesen. Hierbei verwendet man eine markierte Sonde, die sich zwischen den beiden Primem an die nachzuweisende Nukleinsäuresequenz (Templat) anlagert und im Zuge der Primerextension durch die 5'-3'-Exonukleaseaktivität der verwendeten Polymerase abgebaut wird. Durch den Abbau wird ein Signal generiert. Die Detektion dieses Signals kann als Maß der fortschreitenden Amplifikation gewertet werden, da die Stärke des Signals proportional ist zu der Anzahl generierter Amplifikate und der zeitabhängige Signalverlauf darüber hinaus Rückschlüsse auf die Menge an Templat zuläßt. Diese Art von Essay befindet sich unter der Bezeichnung "TaqMan®" im Handel.

Eine weitere Möglichkeit, Nukleinsäuren nachzuweisen, bieten Techniken, die auf einer spezifischen Hybridisierung beruhen. Diese Techniken können prinzipiell auf die in der Probe vorhandenen Nukleinsäuren und davon ableitbare Nukleinsäuren angewendet werden, wobei die Nukleinsäuren bzw. die davon ableitbaren Nukleinsäuren zuvor einer Amplifikation unterworfen werden können oder nicht.

Dem Fachmann sind prinzipiell eine Vielzahl von verschiedenen Hybridisierungsformaten bekannt. Erfindungsgemäß bevorzugt ist die Verwendung von Sonden zum sequenzspezifischen Nukleinsäurenachweis. Dazu geht man in der Regel folgendermaßen vor.

Fällt die nachzuweisende Nukleinsäure in doppelsträngiger Form an, sollte sie zuvor in eine einzelsträngige Form überführt werden. Geeignete Maßnahmen sind dem Fachmann hinlänglich bekannt. So können doppelsträngige Nukleinsäure wie sie beispielsweise durch eine PCR erzeugt werden, denaturierenden Bedingungen unterworfen werden, wie erhöhter Temperatur, hoher lonenstärke und/oder alkalischem pH-Wert.

Zur Hybridisierung lässt man die Hybridisierungskomponenten unter Bedingungen, die eine Duplex-Bildung zwischen nachzuweisender Nukleinsäuresequenz und dazu komplementärer Sonde zulassen, aufeinander einwirken. Beispielsweise bringt man die immobilisierte Sonde in Kontakt mit einem Hybridisierungsgemisch, welches die Nukleinsäure oder eine davon ableitbare Nukleinsäure, und gegebenenfalls weitere übliche Zusätze umfaßt. Es versteht sich, daß Teile des Gemisches zunächst getrennt voneinander mit der Sonde in Kontakt gebracht werden können. Die Hybridisierungsbedingungen werden zweckmäßigerweise so gewählt, daß Sonde und dazu komplementäres Target stabile Hybride bilden können. In der Regel werden zunächst Bedingungen relativ niedriger Stringenz gewählt, z.B. Temperaturen von etwa 20 - 50°C sowie lonenstärken von etwa 6 x SSPE oder niedriger. Anschließend kann dann bei ähnlicher oder höherer Stringenz, z.B. etwa 2 x SSPE bis etwa 0,1 x SSPE bei etwa 30 - 50°C gewaschen werden. Ferner kann auf bekannte Agenzien, z.B. Detergenzien, Blockreagenzien, denaturierende Agenzien, die Renaturierung beschleunigende Agenzien und Tm-egalisierende Agenzien zurückgegriffen werden. Die Optimierung des Hybridisierungsprotokolls ist Sache des Fachmanns.

Vorzugsweise verwendet man zur Hybridisierung immobilisierte Sonden. Hierzu sind die Sonden an einen Träger gekoppelt, beispielsweise über kovalente, adsorptive oder über physikalisch/chemische Wechselwirkungen von Sonde und Oberfläche. Geeignete Methoden zur Erzielung einer zweckmäßigen Kopplung sind dem Fachmann bekannt. Dabei kann die zuvor bereitgestellte Sonde an die Oberfläche gekoppelt werden oder es kann die Sonde insitu an der Oberfläche synthetisiert werden, z.B. mittels photolitographischer Verfahren. Die Kopplung über photoaktive Gruppen, beispielsweise bestimmte Anthrachinone, und erforderlichenfalls einen Spacer geeigneter Länge und Konstitution, beispielsweise Polyethylenglykol mit n = 2-10 und vorzugsweise von etwa 6 bei 8-60meren, an eine reaktive Oberfläche, stellt eine bevorzugte Ausführungsform dar. Arrays immobilisierter Sonden sind als handliches und leistungsstarkes Format besonders bevorzugt. Auf die diesbezüglichen Ausführungen in WO 02/18634 wird vollumfänglich Bezug genommen.

Der sequenzspezifische Nachweis basiert in der Regel auf der Bestimmung, ob Sonde und Nukleinsäure einen Duplex bilden, d.h. miteinander hybridisieren. Somit beinhaltet der erfindungsgemäße Nachweis, ob eine bestimmte Target-Sequenz vorhanden ist oder nicht (Anwesenheit oder Abwesenheit). Die Feststellung soll in Bezug auf die Expressionsprodukte der Gene MNSOD, TXNRD und GPX quantitativ erfolgen.

In der Regel erfordert der Nachweis eine Quantifizierung derjenigen Nukleinsäuren, die an eine immobilisierte Sonde hybridisieren. Die Quantifizierung kann absolut oder relativ erfolgen. Geeignete Detektionssysteme sind dem Fachmann hinlänglich bekannt. Eine vielfach genutzte Möglichkeit besteht in der Einführung von Markierungen, z.B. radioaktiver, colorimetrischer, fluoreszierender oder lumineszierender Art. Diese werden erfindungsgemäß bevorzugt im Zuge einer der Hybridisierung vorgeschalteten Amplifikation - -wie bereits oben erläutert - eingeführt, oder bei indirekten Markierungen nach Einführung eines primären Labels wie Biotin oder Digoxigenin (DIG) durch Zugabe von entsprechenden Markern wie Fluoreszenz-markiertem oder POD-markiertem Streptavidin bzw. anti-DIG sowie, im Falle von Chemi- oder Biolumineszenz, üblicherweise durch Zugabe von Enzymsubstratlösungen oder, wenn Substratmoleküle wie Luminol als Marker verwendet werden, von Enzymlösungen, nachgewiesen.

Aus der Vielzahl geeigneter Detektionssysteme kann der Fachmann insbesondere die in WO 02/08458 beschriebenen photoelektrischen Fächensensoren, insbesondere CCD-Cameras oder die in DE 100 38 080 beschriebenen Fluoreszenzscanner wählen.

### c) Auswertung

Mit den zuvor beschriebenen Messmethoden gelingt es, jeder untersuchten Probe einen bestimmten Wert zuzuordnen, der die Expression des untersuchten Gens kennzeichnet. Erfindungsgemäß von besonderer Bedeutung ist die Feststellung, ob die Expression in Zellen der untersuchten Probe vergleichsweise erhöht ist, da eine erhöhte MNSOD-, TXNRD- und GPX-Expression krebsrelevant ist. Daher beinhaltet die erfindungsgemäße Auswertung in der Regel einen Vergleich mit Zellen, in denen keine mit Krebs in Zusammenhang stehende Modifikation zu erwarten ist (Nichtkrebszellen, Normalzellen). Ist die erfindungsgemäße Untersuchung beispielsweise auf Krebszellen in Körperflüssigkeiten gerichtet, wird man als Vergleich Zellen wählen, die in dieser Körperflüssigkeit üblicherweise vorkommen. Im Falle von Blut sind dies insbesondere Zellen des weißen Blutbildes, die man beispielsweise mit Dichtegradienten-Zentrifugation gewinnen (z.B. den Buffy-Coat; die MNC-Fraktion) oder über spezifischere Isolationsverfahren abtrennen kann (z.B. CD45-positive Lymphozyten). Diese Zellen des weißen Blutbildes können prinzipiell auch als Vergleichszellen bzw. Vergleichszellgemische bei der Untersuchung anderer Körperflüssigkeiten als Blut dienen. Eine weitere Möglichkeit besteht darin, zumindest eine zellhaltige Fraktion der Probe, insbesondere der Körperflüssigkeit, durch Maßnahmen zur Anreicherung von Krebszellen in wenigstens zwei Teilfraktionen aufzutrennen, von denen die eine, gegebenenfalls an Krebszellen angereicherte Fraktion als Testzellgemisch, und die andere, gegebenenfalls an Krebszellen abgereicherte Fraktion als Vergleichszellgemisch verwendet werden kann.

Wird die erfindungsgemäße Untersuchung an Zellgemischen durchgeführt, so ist es nicht erforderlich, dass diese Gemische ausschließlich Krebszellen bzw. ausschließlich Nichtkrebszellen beinhalten. Von Bedeutung ist vielmehr das Verhältnis der Zelltypen in den Gemischen zueinander. Für das erfindungsgemäße Verfahren ist es bereits ausreichend, wenn der aufgrund der Maßnahmen zur Gewinnung der Gemische zu erwartende Krebszellanteil in dem Testzellgemisch signifikant höher liegt als in dem Vergleichszellgemisch. So kann das Vergleichszellgemisch durchaus Krebszellanteile aufweisen, sofern der Krebszellanteil im Testzellgemisch hinreichend höher ist. Dies kann beispielsweise dadurch gewährleistet sein, dass das Testzellgemisch aus dem Vergleichszellgemisch durch Maßnahmen zur Anreicherung von Krebszellen gewonnen wird.

Gemäß einer bevorzugten Ausführungsform der vorliegenden Erfindung gewinnt man daher unter Anreicherung von Krebszellen eine erste zellhaltige Fraktion aus der biologischen Probe und bestimmt die Expression der Gene in der zellhaltigen Fraktion, stellt eine weitere zellhaltige Fraktion der biologischen Probe oder einer vergleichbaren biologischen Probe bereit und die bestimmt Expression der Gene in der weiteren zellhaltigen Fraktion, und vergleicht für jedes Gen dessen Expression in der zellhaltigen Fraktion mit dessen Expression in der weiteren zellhaltigen Fraktion. Von Vorteil ist, wenn die vergleichbare biologische Probe aus dem Individuum stammt, dessen biologische Probe auf Krebszellen untersucht wird, d.h. ein Vergleich mit patienteneigenen Nichtkrebszellen vorgenommen wird. Dies ist insbesondere dann von Bedeutung, wenn der betroffende Patient bereits therapeutische Maßnahmen erhalten hat, die sich auf Phäno- und Genotyp seiner Zellen auswirken.

Das erfindungsgemäße Testprinzip basiert daher auf der Feststellung, ob eine Anreicherung von Krebszellen mit einem messbaren Anstieg der MNSOD-, TXNRD- und GPX-Expression verbunden ist. Maßgeblich ist daher das Verhältnis der im Testzellgemisch gemessenen Expression zur im Vergleichszellgemisch gemessenen Expression.

Zweckmäßigerweise wird man zur Validierung eines bestimmten Testsystems in der Regel einen bestimmten Quotienten (Grenzwert) festlegen, ab dem definitionsgemäß eine Überexpression vorliegt.

Dieser Grenzwert kann von den verwendeten Zellgemischen und insbesondere von deren Gewinnung abhängen. So ist es zweckmäßig, eine bestimmte Ausführungsform des erfindungsgemäßen Verfahrens zunächst an gesunden, d.h. nicht an Krebs erkrankten Individuen durchzuführen und mit Hilfe statistischer Methoden einen geeigneten Grenzwert für die verwendete Ausführungsform des Verfahrens festzusetzen. So kann man unter Berücksichtigung statistischer Signifikanz das Verfahren an einem hinreichend großen Kollektiv von Individuen durchführen, aus den gemessenen Expressionsverhältnissen den Mittelwert bilden und den Grenzwert unter Berücksichtigung des Mittelwerts und der dazugehörigen Standardabweichung festlegen. Ein auf diese Art und Weise ermittelter Grenzwert berücksichtigt beispielsweise auch diejenigen Fälle, in denen das Testzellgemisch im Vergleich zum Vergleichszellgemisch eine verstärkte Expression des gemessenen Parameters zeigt, obwohl auch das Testzellgemisch keine Krebszellen enthält. Derartige Fälle können insbesondere dann auftreten, wenn das eigentlich zur Anreicherung von Krebszellen gewählte Verfahren zur Anreicherung von Nichtkrebszellen führt, die ebenfalls eine verstärkte Expression des gemessenen Parameters zeigen. Dieser Fall konnte beispielsweise im Hinblick auf die GPX1-Expression beobachtet werden, wenn man die Zellen des weißen Blutbildes einem größen- und gestaltabhängigen Trennvorgang unterwarf. Darüber hinaus ist es in der Regel zweckmäßig, die an den Testzellgemischen bzw. Vergleichszellgemischen gemessenen Werte auf einen Standard zu beziehen. Ein solcher Standard lässt sich beispielsweise mit Hilfe von Zelllinien erstellen, die eine hinreichend starke Expression des zu bestimmenden Genexpressionsproduktes zeigen (Positivkontrolle). Beispielsweise eignen sich die Mammakarzinom-Zelllinie EFM 192 als Positivkontrolle für die Bestimmung der MNSOD- und GPX1-Expression, und die Mammakarzinom-Zelllinie MES-SA/Dx5 beispielsweise als Positivkontrolle für die TXNRD1-Expression. Weitere geeignete Referenzzelllinien sind entweder bekannt oder können mit dem erfindungsgemäßen Verfahren etabliert werden, wie z.B. die Mammakarzinom-Zelllinie BT474.

Die erfindungsgemäße Untersuchung auf Krebszellen beinhaltet insbesondere deren Identifizierung und/oder deren Charakterisierung.

Ein weiterer Gegenstand der vorliegenden Erfindung ist daher die Verwendung des erfindungsgemäßen Verfahrens zur Identifizierung von Krebszellen, insbesondere in der Tumorfrüherkennung. Damit verbunden ist insbesondere die analytische Feststellung, ob die untersuchte Probe Krebszellen aufweist, bzw. die diagnostische Feststellung, ob das Individuum, dessen Probe untersucht wurde, an Krebs erkrankt ist. Eine erhöhte Expression wenigstens eines MNSOD-Gens in Kombination mit einer erhöhten Expression wenigstens eines TXNRD-Gens und/oder wenigstens eines GPX-Gens, und insbesondere eine erhöhte Expression wenigstens eines MNSOD-Gens in Kombination mit einer erhöhten Expression wenigstens eines TXNRD-Gens in Kombination mit einer erhöhten Expression wenigstens eines GPX-Gens ist ein als Hinweis auf das Vorliegen von Krebszellen in der untersuchten Proben zu werten.

Hierzu gehören insbesondere der Nachweis von Tumorzellen aus Sputum/Speichel, vor allem zur Früherkennung von Lungentumoren; aus Urin, vor allem zur Früherkennung von Prostata- und Blasentumoren; aus Stuhl, vor allem zur Früherkennung von Colon- und Pankreastumoren; aus Blut/Knochenmark/Lymphe, vor allem zur Früherkennung von allen streuenden Tumoren.

Ein Gegenstand der vorliegenden Erfindung ist auch die Verwendung des erfindungsgemäßen Verfahrens zur Charakterisierung von Krebszellen, z.B. zur Klassifizierung von Tumoren und zur Risikoabschätzung für den Patienten. Damit verbunden sind prognostische Feststellungen über den zukünftigen Verlauf einer Krebserkrankung, wie die Wahrscheinlichkeit (Risiko), eine Metastase oder ein Rezidiv zu entwickeln bzw. eine bestimmte Zeit zu überleben, und therapeutische Feststellungen über die Wirksamkeit einer angewendeten Therapie (Therapie-Monitoring) oder Feststellungen zur Therapiewahl. Eine erhöhte Expression wenigstens eines MNSOD-Gens in Kombination mit einer erhöhten Expression wenigstens eines TXNRD-Gens und/oder wenigstens eines GPX-Gens, und insbesondere eine erhöhte Expression wenigstens eines MNSOD-Gens in Kombination mit einer erhöhten Expression wenigstens eines TXNRD-Gens in Kombination mit einer erhöhten Expression wenigstens eines GPX-Gens ist mit einem erhöhten Risiko, eine Metastase oder ein Rezidiv zu entwickeln bzw. mit einer verringerten Wahrscheinlichkeit, eine bestimmte Zeit zu überleben, verbunden. Um die Wirksamkeit einer angewendeten Therapie (Therapie-Monitoring) zu beurteilen, wird das erfindungsgemäße Verfahren zumindest an zwei verschiedenen Zeitpunkten, d.h. vor und nach einer bestimmten therapeutischen Maßnahme, durchgeführt. Durch einen Vergleich der vor und nach der Maßnahme bestimmten Expression lässt sich feststellen, ob die therapeutische Maßnahme zu einer Veränderung der Anzahl der in der Probe mit dem erfindungsgemäßen Verfahren identifizierbaren Krebszellen geführt hat. Eine Abnahme ist ein Hinweis auf die Wirksamkeit der therapeutischen Maßnahme. Es lassen sich auf diese Art insbesondere solche therapeutische Maßnahmen beurteilen, die eine Verringerung oder Eliminierung disseminierter Krebszellen bewirken sollen.

Gemäß einer besonderen Ausführungsform ist das erfindungsgemäße Verfahren Teil einer Multiparameter-Untersuchung, die zusätzlich zu den drei erfindungsgemäßen Genexpressionsanalysen die Untersuchung weiterer Parameter mit einbezieht. Im Prinzip sollten so viele Parameter wie möglich untersucht werden, so dass eine Beschränkung in der Regel lediglich aus Gründen der Zweckmäßigkeit und Praktikabilität erfolgt. In der Regel werden im Rahmen solcher Multiparameter-Untersuchungen bis zu 10000, insbesondere bis zu 1000, vorzugsweise bis zu 100, besonders bevorzugt bis zu 75, 50 oder 25 und insbesondere bis zu 10 Parameter untersucht.

Der Begriff "Parameter" bezeichnet in diesem Zusammenhang jedwede biochemische oder molekularbiologische Eigenart von Krebszellen und insbesondere von disseminierten Krebszellen. Hierzu gehören sowohl therapeutische als auch diagnostische, insbesondere prognostische Parameter. Genomische Parameter, z.B. mit Ausprägung auf DNA-Ebene zählen genauso dazu wie Parameter aus dem Expressionsbereich, z.B. solche mit Ausprägung auf RNA-, insbesondere mRNA- oder Proteinebene. Beispielsweise können Mutationen, Insertionen, Deletionen, LOHs, Amplifikationen, Aberrationen im Chromosomensatz und dgl.; die Expression von Splice-Varianten; sowie die Über- und Unterexpression bestimmter mRNAs bzw. Proteine - und weitere ungewöhnliche, insbesondere krebsspezifische Veränderungen bestimmter Zellbestandteile als Parameter dienen.

Bevorzugt werden Parameter, die qualitative Eigenarten des DNA- und/oder RNA-Apparates betreffen. Vor allem sind hier Parameter zu nennen, die Zelleigenschaften wie die Zellteilung, das Zellwachstum, Zell-Zell-Interaktionen, Inhibition der Tumorsuppression und Therapieresistenzen betreffen und insbesondere onkogen beeinflussen und damit das klinische Bild einer Krebserkrankung mitbestimmen. Parameter aus dem Bereich der DNA-Rekombination, DNA-Amplifikation, DNA-Reparatur, ZeitzyMusinduktoren und Apoptoseinhibitoren zählen insbesondere dazu.

Beispielhaft seien genannt:
- vor allem Onkogene bzw. Tumorsuppressorgene, wie p53, Gene der ras-Familie, erb-B2, c-myc, mdm2, c-fos, DPC4, FAP, nm23, RET, WT1 u.ä., LOH's, beispielsweise im Hinblick auf p53, DCC, APC, Rb u.ä. sowie BRCA1 und BRCA2 bei heriditären Tumoren, Mikrosatelliten-Instabilität von MSH2, MLH1, WT1 u.ä.; auch tumoröse RNA's, wie CEA, Cytokeratine, z.B. CK20, BCL-2, MUC1, insbesondere tumorspezifische Splice-Varianten hiervon, MAGE3, Muc18, Tyrosinase, PSA, PSM, BA46, Mage-1 u.ä., oder ferner morphogene RNA's, wie Maspin, HCG, GIP, Motilin, hTG, SCCA-1, AR, ÖR, PR, verschiedene Hormone u.ä., betreffen;
- weiterhin vor allem RNA's und Proteine, die das Metastasierungsprofil, d.h. die Expression von Angiogenese-, Motilitäts-, Adhäsions- und Matrixdegradationsmolekülen, wie bFGF, bFGF-R, VEGF, VEGF-R's, wie VEGF-R1 oder VEGF-R2, E-Cadherin, Integrine, Selectine, MMP's, TIMP's, SF, SF-R u.ä., das Zellzyklus- bzw. Proliferationsprofil, wie Cycline (z.B. das Expressionsverhältnis von Cyclin D, E und B), Ki67, P120, p21, PCNA u.ä., oder das Apoptose-Profil, wie FAS (L+R), TNF (L+R), Perforin, Granzyme B, BAX, bcl-2, Caspase 3 u.ä. , betreffen.

Diese und weitere Parameter werden in der WO 99/10528, der WO 00/06702 sowie in Giesing M. et al., The International Journal of Biological Markers Bd. 15(1), 94-99, 2000 beschrieben und erläutert.

Auch betrifft die vorliegende Erfindung Analysekits zur Durchführung des erfindungsgemäßen Verfahrens. Diese enthalten in der Regel
i) wenigstens ein Mittel zur Bestimmung der MNSOD-Genexpression, insbesondere spezifische Antiköper oder vorzugsweise sequenzspezifische Primer und/oder Sonden, wie die oben beschriebenen, z.B. Primer mit den Sequenzen SEQ ID NO:1 und/oder SEQ ID NO:2 bzw. Sonden mit der Sequenz SEQ ID NO:3;
ii) wenigstens ein Mittel zur Bestimmung der TXNRD1-Genexpression, insbesondere spezifische Antiköper oder vorzugsweise sequenzspezifische Primer und/oder Sonden, wie die oben beschriebenen, z.B. Primer mit den Sequenzen SEQ ID NO:4 und/oder SEQ ID NO:5 bzw. Sonden mit der Sequenz SEQ ID NO:6; und/oder
iii) wenigstens ein Mittel zur Bestimmung der GPX1-Genexpression, insbesondere spezifische Antiköper oder vorzugsweise sequenzspezifische Primer und/oder Sonden, wie die oben beschriebenen, z.B. Primer mit den Sequenzen SEQ ID NO:7 und/oder SEQ ID NO:8 bzw. Sonden mit der Sequenz SEQ ID NO:9; sowie gegebenenfalls
iv) weitere übliche Mittel zur Durchführung des erfindungsgemäßen Verfahrens.

Weitere besondere Ausführungsformen erfindungsgemäßer Kits ergeben sich aus den Ausführungen zum Verfahren selbst.

Weiterhin ermöglicht die vorliegende Erfindung ein Verfahren zum Testen und/oder zur funktionellen Validierung von Wirkstoffen. Dazu läßt man den Wirkstoff in der Regel ex vivo auf disseminierte Krebszellen, die durch eine erhöhte Expression der Gene MNSOD, TXNRD1 und/oder GPX1 gekennzeichnet sind, einwirken und bestimmt deren Reaktion, insbesondere die nach der Einwirkung des Wirkstoffs resultierende Expression der Gene MNSOD, TXNRD1 und/oder GPX1. Zur Kontrolle können die Verfahrensmaßnahmen in entsprechender Weise an Zellen vorgenommen werden, deren Expression der Gene MNSOD, TXNRD1 und/oder GPX1 nicht erhöht ist. Es kann in der Regel auf an sich bekannte zellbiologische Testsysteme zurückgegriffen werden. Erforderlichenfalls können disseminierte Krebszellen in Kultur gehalten und geeignete Bioassays durchgeführt werden. Beispielsweise kann man so bekannte Wirkstoffe mit antineoplastischer Wirkung und/oder zur adjuvanten Therapie eingesetzte Wirkstoffe testen. Insbesondere können targetbezogene Wirkstoffe getestet werden. Erfindungsgemäß kann es sich bei diesen Targets insbesondere um die Gene MNSOD, TXNRD1 und/oder GPX1 bzw. deren Expressionsprodukte handeln. Es sind aber auch andere Targets denkbar, die in einem funktionellen Zusammenhang mit besagten Genen bzw. deren Expressionsprodukten stehen und daher in Bezug auf die MNSOD-, TXNRD1- und/oder GPX1-Genexpression validiert werden können. Dies ist ein wichtiger Aspekt der Wirkstoffentwicklung, wonach potentielle Wirkstoffe targetbezogen - beispielsweise im Rahmen von Screening-Verfahren - ausgewählt und anschließend validiert werden können.

Zweck dieses Verfahrens zur funktionellen Validierung von Wirkstoffen ist es, wirkstoffabhängige, molekulare und/oder morphologische Veränderungen an den disseminierten Krebszellen festzustellen. Ergibt die Bestimmung eines oder mehrerer Parameter an den disseminierten Krebszellen einen Zustand, der nach Einwirken des Wirkstoffs von dem Zustand abweicht, der vor Einwirken des Wirkstoffs bestand, ist das Target bezüglich des Wirkstoffs bzw. der Wirkstoff bezüglich des Targets erfindungsgemäß funktionell validiert. Die funktionelle Validierung erfasst insbesondere einen funktionellen Zusammenhang zwischen Wirkstoff und Target in disseminierten Krebszellen.

Gegebenenfalls kann die erfindungsgemäße funktionelle Validierung an disseminierten Krebszellen auf einer funktionellen Prävalidierung des Targets an anderen Zellsystemen aufbauen. Beispielsweise können Targets in an sich bekannter Weise kloniert und exprimiert werden. Dem Fachmann stehen hierzu geeignete Zellsysteme, insbesondere humane Zellinien zur Verfügung, die entsprechend transfiziert werden können. Derartige targetaufweisende Zellsysteme können in der bereits oben beschriebenen Art und Weise mit einem oder mehreren Wirkstoffen in Kontakt gebracht werden. Auch dieses Verfahren dient zur Etablierung eines molekularen und/oder morphologischen Wirk-Algorithmus, der wiederum erfindungsgemäß an disseminierten Krebszellen validiert werden kann.

Dieses Verfahren bietet eine vorteilhafte Grundlage für die Entwicklung und Testung targetbezogener Wirkstoffe. Die Ausrichtung auf klinisch und funktionell einheitlich an disseminierten Krebszellen validierte Targets gestattet eine Wirkstoffentwicklung unter Einbezug pharmako- und toxikogenomischer Aspekte zur Verringerung unerwünschter Nebenwirkungen und einer korrekten Stratifizierung von Patienten, d.h. einer - erforderlichenfalls zeitabhängig - individualisierten Anwendung von Wirkstoffen. Im Vergleich zu herkömmlichen Wirkstoffentwicklungen ergibt sich eine erhebliche Kosten- und Zeitersparnis.

Weiterhin ermöglicht die vorliegende Erfindung die Behandlung von Krebs durch Modulation der Expression von wenigstens zwei Genen, die ausgewählt sind unter Mangan-Superoxiddismutase-Genen, Thioredoxinreduktase-Genen und Glutathionperoxidase-Genen.

Insbesondere ist es Zweck der erfindungsgemäßen Behandlung, die Expression dieser Gene zu verringern. Dabei richtet sich die Behandlung vornehmlich auf die zellulären Bestandteile von Körperflüssigkeiten, in denen zuvor disseminierte Krebszellen diagnostiziert wurden. Es ist davon auszugehen, dass durch die erfindungsgemäße Behandlung der Phänotyp eines Großteils der in einem Individuum vorhandenen disseminierten Krebszellen derart beeinflusst wird, dass sich Überlebensfähigkeit der disseminierten Krebszellen verringert.

Ohne an einen bestimmten Mechanismus gebunden zu sein, kann ein solcher Behandlungseffekt damit erklärt werden, dass die disseminierten Krebszellen einen durch die Überexpression eines oder mehrerer obiger Gene vermittelten Schutzmechanismus verlieren bzw. den in der jeweiligen Körperflüssigkeit herrschenden Bedingungen weniger angepasst sind, so dass es zu einer verstärkten Eliminierung der disseminierten Krebszellen kommt.

Eine vorteilhafte Behandlungsvariante richtet sich auf die Modulation der MNSOD-Expression in Kombination mit einer Modulation der TXNRD- und/oder GPX-Expression. Besonders vorteilhaft ist die Modulation der MNSOD-, TXNRD- und GPX-Expression.

Verfahren und Mittel zur Expressionsmodulation bestimmter Gene sind im Prinzip bekannt. Insbesondere die Verringerung der Genexpression kann beispielsweise auf RNA-Ebene mit Hilfe spezifischer Antisense-Moleküle erreicht werden. Auf Protein-Ebene wird die Expressionsverminderung mit Hilfe spezifischer Bindungspartner erreicht werden, die eine hinreichende Affinität für die exprimierten Proteine besitzen und diese in ihrer Funktion beeinträchtigen. Hierzu gehören beispielsweise spezifische Antikörper, aber auch niedermolekulare Verbindungen, die im vorliegenden Fall in Anlehnung an die von den Enzymen katalysierten Umsetzungen und insbesondere den umgesetzten Substraten entwickelt werden können. Demnach können insbesondere Inhibitoren der MNSOD-, TXNRD- und/oder GPX-Aktivität eingesetzt werden.

Es wird daher einem zu behandelnden Individuum eine wirksame Menge einer Wirkstoffkombination verabreicht, welche die Expression von wenigstens zwei unter MNSOD-, TXNRD- und GPX-Genen ausgewählten Genen zu vermindern bzw. disseminierte Krebszellen des behandelten Individuums zu eliminieren vermag.

Die vorliegende Erfindung ermöglicht daher auch die Verwendung einer Kombination entsprechender Wirkstoffe zur Bereitstellung eines pharmazeutischen Mittels zur Behandlung von Krebs. Dabei kann diese Wirkstoffkombination in Form eines entsprechenden Wirkstoff-Cocktails oder in Form von Einzelwirkstoffen zu unterschiedlichen Zeitpunkten, beispielsweise abwechselnd zu unterschiedlichen Tageszeitpunkten oder sequenziell, verabreicht werden, wobei die Wirkstoffe in der Regel der pharmazeutischen Praxis entsprechend als pharmazeutisches Mittel zubereitet sind.

### Ausführungsbeispiele

### Figurbeschreibung

Figur 1 zeigt die als Balkendiagramm aufgetragene Auswertung einer CCD-Kontaktbelichtungsaufnahme der von einem mit den benannten genspezifischen Sonden bestückten Array emittierten Fluoreszenzstrahlung nach Hybridisierung mit cDNA-Einzelstrangfragmenten, die aus Zellen im Blut eines Tumorpatienten gewonnen wurden.

### Proben

Folgende Proben werden für die nachstehend beschriebenen Untersuchungen herangezogen:
Blut aus 9 gesunden Spendern und 47 Tumor-Patienten;
Mammakarzinom-Zelllinie BT474 (Referenzzelllinie für eine MNSOD-, TXNRD1- und GPX1-Überexpression)

### Tumorzell-Isolierung (Krebszellfraktion C)

10 ml heparinisiertes Blut werden abzentrifugiert (400 g; 10 min; RT). Das überstehende Plasma wird abgenommen. Die pelletierten Zellen werden in 12 ml PBS aufgenommen. Nach Dichtegradientenzentrifugation (Nycodenz 1.077; 800 g; 30 min, RT) werden die Interphasezellen (im Wesentlichen mononukleäre Zellen, kurz MNC-Fraktion) abgenommen und 2 x in 10 ml PBS (1 mM EDTA) gewaschen (400 g; 10 min; 4°C). Die MNC-Fraktion wird in 10 ml PBS ausgenommen (1 mM EDTA, 0,5 % BSA). Als mögliche Bezugsgröße wird 1 ml dieses Zellgemisches abgenommen (Vergleichsfraktion A'). Die restlichen 9 ml Zellgemisch werden über eine Säule durch ein aus Polyester-Fäden gewebtes Sieb mit 20 µm Maschenweite (vertrieben von der SEFAR AG, Rüschlikon, Schweiz) geführt, und der Siebdurchlauf wird als mögliche Bezugsgröße gesammelt (Vergleichsfraktion B'). Die Säule wird 5 x mit je 10 ml PBS (1 mM EDTA) gewaschen. Das Sieb wird herausgenommen, umgedreht und in einem Reaktionsgefäß mit 0,7 ml Trizol® inkubiert (5 min; RT). Das Sieb wird im Reaktionsgefäß oberhalb der Trizol®-Lösung plaziert und abzentrifugiert (200 g; 30 s; RT). Das trockene Sieb wird entfernt und die Trizol® -Lösung (Krebszellfraktion C) der weiteren RNA -Isolation zugeführt.

Alternativ zur Inkubation des Siebes in Trizol® kann das Sieb der Säule entnommen, umgedreht und in PBS (1 mM EDTA, 0,5 % BSA) überführt werden, und die Zellen können durch Zentrifugation (400 g; 10 min, 4°C) pelletiert und der weiteren RNA-Isolation zugeführt werden.

### Normalzell (Nichtkrebszell)-Isolierung (Vergleichsfraktionen A und B)

Zur Isolierung von CD45-positiven Lymphozyten als Vergleichsfraktionen werden jeweils 1/10 der MNC-Fraktion vor (Fraktion A') bzw. nach (Fraktion B') dem Siebvorgang abgenommen. Diese werden in ein 1 ml PBS (0.5% BSA, 100 µg hu-IgG) enthaltendes Reaktionsgefäß überführt. Dazu werden 50 µl gewaschene anti-CD45-Microbeads gegeben. Der Ansatz rotiert 20 min bei 4°C. Anschließend wird das Reaktionsgefäß so an einer Magnetleiste positioniert, dass die Microbeads (gebunden an CD45-positive MNC's) an der Gefäßwand pelletieren. Durch dreimaliges Waschen der Bead-Zell-Aggregate erhält man eine reine Population CD45-positiver Lymphozyten, welche dann in Trizol® gelöst der RNA-Isolation zugeführt werden kann. CD45-lsolate der MNC-Fraktion vor und nach dem Siebvorgang werden als Vergleichsfraktion A bzw. B bezeichnet.

### RNA-Isolierung

Die Extraktion und Aufreinigung der RNA aus obigen Zelllinien erfolgt in an sich bekannter Weise, z.B. mit Hilfe geeigneter Kits wie sie von kommerziellen Anbietern, z.B. den Firmen Qiagen und GIBCO-BRL erhältlich sind.

### mRNA-Expressionsanalyse

Der Mengen exprimierter mRNA werden mittels quantitativer RT-PCR bestimmt. Das PCR-Format basiert auf dem an sich bekannten 5'-Exonuklease-Assay (z.B. TaqMan®) und ist für die Anwendung auf dem Sequence Detector 7700 TaqMan® der Firma Applied Biosystems (ABI) geeignet.

Folgende Reagenzien werden eingesetzt:
a) Reverse Transkription (RT)
   5x First Strand-Puffer (Fa. Boehringer)
   0,1 M Dithiothreitol (DTT)
   RNA-Guard 38950U/ml (Fa. Pharmacia)
   Random Hexamere 500µg/ml (Fa. Promega)
   dNTPs je 20mM (Fa. Pharmacia)
   M-MLV 200U/µl (Fa. Gibco)
b) PCR
   10x TaqMan®-Puffer (Fa. Perkin Elmer (PE))
   MgCl₂ 25 mM (PE)
   dNTP-Mix (je 0,75 µl; 2,5mM)
   ROX-Lösung (100x) (TIB)
   Amplitaq®-Gold (PE) (für Hot Start-Methode)

Die genannten Reagenzien sind im Perkin Elmer TaqMan® PCR Core Reagent Kit enthalten.

Zur Durchführung der RT-PCR:

### 1. Die Reverse Transkription (RT)

Zunächst wird ein RT-Mix aus 2,35 µl H₂O, 4 µl 5x First Strand-Puffer, 2 µl 0,1 M DTT 0,15 µl RNA-Guard (38950 U/ml), 0,5 µl Random-Hexamere (500 µg/ml), 0,5 µl dNTP-Mix zu je 20mM und 0,5 µl M-MLV (200 U/µl) vorbereitet.

Dann werden 10 µl RNA (ca. 1µg aus RNA-Isolierung) 1 min bei 70°C denaturiert, sofort auf Eis gestellt und 3 min abgekühlt, anschließend mit 10 µl RT-Mix luftblasenfrei vermischt, zunächst 60 min bei 37°C und dann 3 min bei 95°C inkubiert, sofort auf Eis gestellt und 3 min abgekühlt.

Dieses das reverse Transkriptat (cDNA) enthaltende Reaktionsgemisch wird entweder direkt der PCR unterworfen oder bei -20°C einfroren.

### 2. Die PCR

Zunächst wird ein PCR-Prämix aus 28,5 µl H₂O, 5,0 µl Puffer (PE), 6µl ROX-Lösung (100x) (TIB), 6,0 µl MgCl₂ (25mM), 3,0 µl dNTP-Mix (je 0,75 µl; 2,5mM), 1,0 µl Primer (sense; 20 pmol/µl). 1,0 µl Primer (antisense; 20 pmol/µl), 0,5 µl Sonde (20 pmol/µl) und 0,5 µl Amplitaq-Gold (PE 5U/ µl) vorbereitet.

Für die Detektion der PCR-Produkte auf dem Sequence Detector 7700 TaqMan® sind spezielle Reaktionsgefäße erforderlich. Geeignet sind die PE Optical Tubes in Kombination mit den PE Optical Caps. Pro Tube setzt man 47µl PCR-Prämix sowie 3µl cDNA-Lösung ein.

Für die Amplifikation wählt man dann eine 2-Schritt-Methode bei folgenden Thermocycling-Bedingungen:

| | | |
|---|---|---|
| 95° C | 10 min Hot Start Aktivierung | |
| 95° C | 30 sec | |
| 60°C | 60 sec | |
| 20° C | unendlich | Zyklenanzahl: 45 |

### 2.1 Bestimmung der Mangan-Superoxiddismutase-mRNA (MNSOD-mRNA)

Folgende MNSOD-spezifische Primer und Sonden werden verwendet (MNSOD , SOD2; Accession-Nr.: M36693):
sense: 5'- GTCACCGAGGAGAAGTACCAGG -3'(SEQ ID NO:1)
antisense: 5'- GGGCTGAGGTTTGTCCAGAA-3' (SEQ ID NO:2)
Sonde: 5'- CGTTGGCCAAGGGAGATGTTACAGCCC -3' (SEQ ID NO:3)
Grösse des PCR-Produktes: 131 bp.

### 2.2 Bestimmung der Thioredoxinreduktase1-mRNA (TXNRD1-mRNA)

Folgende TXNRD1-spezifische Primer und Sonden werden verwendet (TXNRD1; Accession-Nr.: X91247 cDNA):
sense: 5'- GGAGGGCAGACTTCAAAAGCTAC -3' (SEQ ID NO:4)
antisense: 5'-ACAAAGTCCAGGACCATCACCT -3' (SEQ ID NO:5)
Sonde: 5'- TTGGGCTGCCTCCTTAGCAGCTGCCA -3' (SEQ ID NO:6)
Grösse des PCR-Produktes: 158 bp.

### 2.3 Bestimmung der Glutathionperoxidase-mRNA (GPX1-mRNA)

Folgende GPX1-spezifische Primer und Sonden werden verwendet (GPX1; Accession-Nr.: M21304):
sense: 5'- CTCGGCTTCCCGTGCAA -3' (SEQ ID NO:7)
antisense: 5'- TGAAGTTGGGCTCGAACCC -3' (SEQ ID NO:8)
Sonde: 5'- AGTTTGGGCATCAGGAGAACGCCAAGAA -3' (SEQ ID NO:9)
Grösse des PCR-Produktes: 109 bp .

### 2.4 Bestimmung der Glyceraldehyd-3-phosphatdehydrogenase-mRNA (GAPDH-mRNA)

Folgende GAPDH-spezifische Primer und Sonden werden verwendet (GAPDH; Accession-Nr. X01677):
sense: 5'- TGCTGATGCCCCCATGTTC -3' (SEQ ID NO:10)
antisense: 5'- GGCAGTGATGGCATGGACTG -3' (SEQ ID NO:11)
Sonde: 5'- TCAAGATCATCAGCAATGCCTCCTGCA -3' (SEQ ID NO:12)
Grösse des PCR-Produktes: 174 bp.

### 3. Die Auswertung

Zur Auswertung bildet man für die Fraktionen A bzw. A' und C jeweils das Verhältnis von Zelläquivalenten der zu bestimmenden mRNA zu Zelläquivalenten GAPDH-mRNA und setzt die resultierenden Quotienten wiederum ins Verhältnis. Bei einem Verhältnis des Fraktion C-Quotienten zu dem Fraktion A-Quotienten von mehr als einem zu definierenden Grenzwert, liegt eine Überexpression der betreffenden mRNA vor.

Die Zelläquivalente beziehen auf einen Zellstandard. Dieser Zellstandard wird hergestellt, indem aus einer bekannten Anzahl von Zellen (z.B. 2 x 10⁸) einer Zellsuspension einer Karzinomzelllinie, die den jeweiligen Parameter exprimiert (Zelllinie BT474 für MNSOD, GPX1 und TXNRD1), in der oben beschriebenen Weise mRNA extrahiert und in cDNA umgeschrieben wird. Diese cDNA wird bei jeder quantitativen Analyse in Form einer Verdünnungsreihe (z.B. 6 Verdünnungsstufen) mitgeführt und dient als Bezugssystem.

### Resultate:

### Beispiel 1: Gesunde Spender:

In Tabelle 1 sind für gesunde Spender (Anzahl N) die in den isolierten Zellen (Fraktion C) bestimmten Mengen an MNSOD-, TXNRD1- und GPX1-mRNA angegeben, und zwar im Verhältnis zu den Mengen entsprechender mRNAs in der Vergleichszellfraktion (Fraktion A).

**Tabelle 1: MNSOD-, TXNRD1- und GPX1-mRNA-Expression im Blut gesunder Spender**

| | **N** | **Mittelwert** | **Standardabweichung** | **Grenze** |
|---|---|---|---|---|
| **MNSOD** | 9 | 0.9300 | 0.2891 | 1.2 |
| **TXNRD1** | 9 | 0.9133 | 0.4166 | 1.3 |
| **GPX1** | 8 | 3.6888 | 1.5533 | 5.2 |

Die aus Blut isolierten Zellen zeigen eine leicht erhöhte GPX1-Expression. Die Expression der MNSOD und TXNRD1 ist bezogen auf die Vergleichszellfraktion, also den Lymphozyten, unverändert.

Für die nachfolgende Beurteilung der an Tumorpatienten gemessenen Expressionswerte werden diejenigen Werte als positiv gewertet, die den Durchschnittswert plus Standardabweichung überschritten. Dieser Werte sind als Grenzwert in Tabelle 1 angegeben.

### Beispiel 2: Tumorpatienten

Es werden eine Reihe von Tumorpatienten mit unterschiedlichen Tumoren in die Untersuchung einbezogen. Die Tumore sind durch verschiedene andere medizinische Verfahrens diagnostiziert worden.

### 1. Sensitivität

Nachfolgend ist für die nach Tumorart aufgeschlüsselten Tumor-Patienten (Anzahl N) angegeben, in wieviel Fällen die Expression der MNSOD-, TXNRD1- bzw. GPX1-mRNA in den isolierten Zellen (Fraktion C) im Verhältnis zu den Zellen der Gesamtzellfraktion (Fraktion A') erhöht ist (positiv).

| MNSOD | | | | |
|---|---|---|---|---|
| | | | | |
| Untersuchungen | | N=93 | | |
| Patienten | | N=90 | | |
| | | | | |
| davon Mamma | 41 | (40 positiv) | | |
| Colon | 8 | ( 8 positiv) | | |
| Prostata | 8 | ( 7 positiv) | | |
| Ovar | 8 | ( 5 positiv) | | |
| Lunge | 5 | ( 2 positiv) | | |
| Blase | 4 | ( 3 positiv) | | |
| Leber | 2 | ( 1 positiv) | | |
| Schilddrüse | 2 | ( 2 positiv) | | |
| Andere | 12 | (10 positiv) | → | **78/90 = 87% POSITIV** |
| | | | | |

| TXNRD1 | | | | |
|---|---|---|---|---|
| | | | | |
| Untersuchungen | | N=93 | | |
| Patienten | | N=90 | | |
| | | | | |
| davon Mamma | 41 | (31 positiv) | | |
| Prostata | 9 | ( 6 positiv) | | |
| Colon | 8 | ( 6 positiv) | | |
| Ovar | 7 | ( 3 positiv) | | |
| Lunge | 5 | ( 1 positiv) | | |
| Blase | 4 | ( 3 positiv) | | |
| Leber | 2 | ( 1 positiv) | | |
| Schilddrüse | 2 | ( 1 positiv) | | |
| Andere | 12 | ( 8 positiv) | → | **60/90= 67% POSITIV** |

| GPX1 | | | | |
|---|---|---|---|---|
| | | | | |
| Untersuchungen | | N=89 | | |
| Patienten | | N=86 | | |
| | | | | |
| davon Mamma | 40 | (25 positiv) | | |
| Colon | 8 | ( 6 positiv) | | |
| Prostata | 7 | ( 5 positiv) | | |
| Ovar | 7 | ( 3 positiv) | | |
| Lunge | 4 | ( 2 positiv) | | |
| Blase | 4 | ( 2 positiv) | | |
| Leber | 2 | ( 2 positiv) | | |
| Schilddrüse | 2 | ( 2 positiv) | | |
| Andere | 12 | ( 6 positiv) | → | **53/86 = 62% POSITIV** |
| | | | | |
| MNSOD und TXNRD1 und GPX1 | | | | |
| | | | | |
| Untersuchungen | | N=88 | | |
| Patienten | | N=85 | | |
| | | | | |
| 0 positiv | 6/85 | = 7% | | |
| 1 positiv | 9/85 | = 11 % | | |
| 2 positiv | 33/85 | = 39% | | |
| 3 positiv | 37/85 | = 44% | | |
| | | | → | **in 93% mindestens 1 Gen POSITIV** |

Ein auf die Bestimmung aller 3 Parameter gerichteter Nachweis besitzt daher eine Sensitivität 93%, während die Sensitivität der Einzelnachweise bei 87, 67 bzw. 62% liegt.

### 2. Korrelation untereinander

Zusätzlich zur Sensitivität jedes einzelnen Parameters zeigt sich, dass in 74% (58/78) der Fälle, in denen die MNSOD-Expression erhöht ist, auch eine verstärkte TXNRD1-Expression zu beobachten ist (der Korrelationskoeffizient rho nach Pearson beträgt 0,75). Dabei haben ein Teil, nämlich 65% (49/75), der Patienten mit einem erhöhter MNSOD-Expression auch eine erhöhte GPX1-Expression (vergleiche Tabellen 2a,b,c: Korrelationsanalyse von TXNRD1 bzw. GPX1 bezogen auf MNSOD und von GPX1 bezogen auf TXNRD1).

Die Korrelation zwischen einer erhöhten MNSOD-Expression und einer erhöhten TXNRD1-Expression in disseminierten Krebszellen war nicht zu erwarten, da die beiden Enzyme unterschiedliche Funktionen besitzen.

**Tabelle 2a: Korrelation von TXNRD1 bezogen auf MNSOD**

| | MNSOD negativ | | MNSOD positiv | |
|---|---|---|---|---|
| TXNRD1 negativ | 10/11 | 91% | 20/78 | 26% |
| TXNRD1 positiv | 1/11 | 9% | 58/78 | 74% |

| | | | | |
|---|---|---|---|---|
| p=0.0001 (Pearson-Test) | | | | |

**Tabelle 2b: Korrelation von GPX1 bezogen auf MNSOD**

| | MNSOD negativ | | MNSOD positiv | |
|---|---|---|---|---|
| GPX1 negativ | 7/11 | 64% | 26/75 | 35% |
| GPX1 positiv | 4/11 | 36% | 49/75 | 65% |

| | | | | |
|---|---|---|---|---|
| p=0.10 (Pearson-Test) | | | | |

**Tabelle 2c: Korrelation von GPX1 bezogen auf TXNRD1**

| | TXNRD1 negativ | | TXNRD1 positiv | |
|---|---|---|---|---|
| GPX1 negativ | 12/27 | 44% | 20/58 | 34% |
| GPX1 positiv | 15/27 | 56% | 38/58 | 66% |

| | | | | |
|---|---|---|---|---|
| p=0.38 (Pearson-Test) | | | | |

### 3. Korrelation mit einer bcl-2-Überexpression

Darüber hinaus zeigt sich, dass in 100% (5/5) der Fälle, in denen die TXNRD1-Expression erhöht ist, auch eine verstärkte bcl-2-Expression zu beobachten ist (der Korrelationskoeffizient rho nach Pearson beträgt 0,32 und nach Spearman 0,58; vergleiche Tabellen 3a,b,c: Korrelationsanalyse von MNSOD, TXNRD1 bzw. GPX1 bezogen auf bcl-2).

Dies zeigt, dass eine erhöhte TXNRD1-Expression an der mit einer erhöhten bcl-2-Expression assoziierten Apoptoseblockade beteiligt ist.

**Tabelle 3a: Korrelation von MNSOD bezogen auf bcl-2**

| | bcl-2 negativ | | bcl-2 positiv | |
|---|---|---|---|---|
| MNSOD negativ | 9/41 | 22% | 0/5 | 0% |
| MNSOD positiv | 32/41 | 78% | 5/5 | 100% |

| | | | | |
|---|---|---|---|---|
| p=0.57 (Pearson-Test) | | | | |

**Tabelle 3b: Korrelation von TXNRD1 bezogen auf bcl-2**

| | bcl-2 negativ | | bcl-2 positiv | |
|---|---|---|---|---|
| TXNRD1 negativ | 20/41 | 49% | 0/5 | 0% |
| TXNRD1 positiv | 21/41 | 51% | 5/5 | 100% |

| | | | | |
|---|---|---|---|---|
| p=0.06 (Pearson-Test) | | | | |

**Tabelle 3c: Korrelation von GPX1 bezogen auf bcl-2**

| | bcl-2 negativ | | bcl-2 positiv | |
|---|---|---|---|---|
| GPX1 negativ | 10/39 | 26% | 3/5 | 60% |
| GPX1 positiv | 29/39 | 74% | 2/5 | 40% |

| | | | | |
|---|---|---|---|---|
| p=0.14 (Pearson-Test) | | | | |

### 4. Korrelation mit Tumor-Patienten

Ein Vergleich zwischen den gesunden Spendern und einigen der Tumorpatienten (Tabellen 4a-c) zeigt, dass zwischen der gemessenen erhöhten Expression der Gene MNSOD, TXNRD1 und GPX1 und den Tumorpatienten eine überraschend deutliche Korrelation besteht.

**Tabelle 4a: Vergleich zwischen gesunden Spendern und Tumorpatienten hinsichtlich der MNSOD-Expression**

| | **Gesunde** | **TUMOR** |
|---|---|---|
| N | 9 | 43 |
| Mittelwert | 0.93 | 4.82 |
| Median | 1.01 | 3.62 |

| | |
|---|---|
| Wilcoxon | p=0.0004 |
| Median | p=0.001 |

**Tabelle 4b: Vergleich zwischen gesunden Spendern und Tumorpatienten hinsichtlich der TXNRD1-Expression**

| | **Gesunde** | **TUMOR** |
|---|---|---|
| N | 9 | 44 |
| Mittelwert | 0.91 | 2.18 |
| Median | 0.97 | 1.72 |

| | |
|---|---|
| Wilcoxon | p=0.02 |
| Median | p=0.01 |

**Tabelle 4c: Vergleich zwischen gesunden Spendern und Tumorpatienten hinsichtlich der GPX1-Expression**

| | **Gesunde** | **TUMOR** |
|---|---|---|
| N | 8 | 38 |
| Mittelwert | 3.69 | 18.98 |
| Median | 2.88 | 13.09 |

| | |
|---|---|
| Wilcoxon | p=0.0006 |
| Median | p=0.002 |

### 5. Korrelation mit Rezidiven

Ein weiterer Vergleich innerhalb der Gruppe der Tumorpatienten zwischen denjenigen, bei denen eine Rezidiv aufgetreten ist, und denjenigen, bei denen ein Rezidiv nicht aufgetreten ist, zeigt, dass zwischen der gemessenen erhöhten Expression der Gene MNSOD, TXNRD1 und GPX1 und dem Auftreten von Rezidiven ebenfalls eine überraschend deutliche Korrelation besteht. Dies gilt für sämtliche der untersuchten Tumoren (Tabellen 5a-c) und insbesondere für Patientinnen mit Mamma-Karzinomen (Tabellen 6a-c). Ein überraschender Vorteil des erfindungsgemäßen Verfahrens liegt darin, dass wenigstens 2 und insbesondere alle 3 Parameter besser mit dem klinischen Verlauf einer Krebserkrankung korrelieren, als ein Parameter allein (Tabellen 7a, b).

**Tabelle 5a: Vergleich zwischen rezidivfreien und rezidivierten Tumorpatienten hinsichtlich der MNSOD-Expression**

| | **kein Rezidiv** | **Rezidiv** |
|---|---|---|
| N | 23 | 14 |
| Mittelwert | 3.12 | 6.17 |
| Median | 2.78 | 5.43 |

| | |
|---|---|
| Wilcoxon | p=0.02 |
| Median | p=0.005 |

**Tabelle 5b: Vergleich zwischen rezidivfreien und rezidivierten Tumorpatienten hinsichtlich der TXNRD1-Expression**

| | **kein Rezidiv** | **Rezidiv** |
|---|---|---|
| N | 23 | 14 |
| Mittelwert | 1.48 | 2.32 |
| Median | 1.17 | 2.09 |

| | |
|---|---|
| Wilcoxon | p=0.05 |
| Median | p=0.14 |

**Tabelle 5c: Vergleich zwischen rezidivfreien und rezidivierten Tumorpatienten hinsichtlich der GPX1-Expression**

| | **kein Rezidiv** | **Rezidiv** |
|---|---|---|
| N | 20 | 14 |
| Mittelwert | 13.01 | 23.49 |
| Median | 9.20 | 15.66 |

| | |
|---|---|
| Wilcoxon | p=0.05 |
| Median | p=0.04 |

**Tabelle 6a: Vergleich zwischen rezidivfreien und rezidivierten Mamma-Karzinompatientinnen hinsichtlich der MNSOD-Expression**

| | **kein Rezidiv** | **Rezidiv** |
|---|---|---|
| N | 12 | 4 |
| Mittelwert | 2.92 | 8.71 |
| Median | 2.73 | 6.66 |

| | |
|---|---|
| Wilcoxon | p=0.03 |
| Median | p=0.03 |

**Tabelle 6b: Vergleich zwischen rezidivfreien und rezidivierten Mamma-Karzinompatientinnen hinsichtlich der TXRND1-Expression**

| | **kein Rezidiv** | **Rezidiv** |
|---|---|---|
| N | 12 | 4 |
| Mittelwert | 1.53 | 2.95 |
| Median | 1.43 | 2.84 |

| | |
|---|---|
| Wilcoxon | p=0.06 |
| Median | p=0.26 |

**Tabelle 6c: Vergleich zwischen rezidivfreien und rezidivierten Mamma-Karzinompatientinnen hinsichtlich der GPX1-Expression**

| | **kein Rezidiv** | **Rezidiv** |
|---|---|---|
| N | 11 | 4 |
| Mittelwert | 11.12 | 19.17 |
| Median | 9.10 | 15.66 |

| | |
|---|---|
| Wilcoxon | p=0.13 |
| Median | p=0.02 |

**Tabelle 7a: Vergleich zwischen rezidivfreien und rezidivierten Mamma-Karzinompatientinnen hinsichtlich der MNSOD, TXNRD1 und GPX1-Expression**

| | **kein Rezidiv** | | **Rezidiv** | |
|---|---|---|---|---|
| 0 | 0/18 | 0% | 0/7 | 0% |
| 1 | 2/18 | 11% | 0/7 | 0% |
| 2 | 8/18 | 44% | 3/7 | 43% |
| 3 | 8/18 | 44% | 4/7 | 57% |

**Tabelle 7b: Vergleich zwischen rezidivfreien und rezidivierten Tumorpatienten hinsichtlich der MNSOD, TXNRD1 und GPX1-Expression**

| | **kein Rezidiv** | | **Rezidiv** | |
|---|---|---|---|---|
| 0 | 1/28 | 4% | 3/29 | 10% |
| 1 | 4/28 | 14% | 2/29 | 7% |
| 2 | 11/28 | 39% | 9/29 | 31% |
| 3 | 12/28 | 43% | 15/29 | 52% |

### 6. Korrelation mit DNA-Aberrationen

Auch das Auftreten von DNA-Aberrationen, d.h. genomischen Imbalancen (G.I.) (vgl. Giesing M, et al. Int J Biol Markers, 15, 94-99, 2000) in den isolierten Zellen korreliert deutlich mit einer erhöhten Expression der Gene MNSOD, TXNRD1 und GPX1, wie die in Tabellen 8a-d dargestellten Ergebnisse belegen. Es korreliert die Anzahl genomischer Imbalancen mit der Anzahl der unter MNSOD, TXNRD1 und GPX1 ausgewählten überexprimierten Gene.

**Tabelle 8a: Vergleich zwischen der Abwesenheit und dem Auftreten genomischer Imbalanzen hinsichtlich der MNSOD-Überexpression**

| | **1 G.I.** | | **≥ 2 G.I.** | |
|---|---|---|---|---|
| SOD > 1.2 | 16/19 | 84% | 8/8 | 100% |

**Tabelle 8b: Vergleich zwischen der Abwesenheit und dem Auftreten genomischer Imbalanzen hinsichtlich der TXNRD1-Überexpression:**

| | **1 G.I.** | | **≥ 2 G.I.** | |
|---|---|---|---|---|
| TXNRD1 > 1.3 | 12/19 | 63% | 5/9 | 56% |

**Tabelle 8c: Vergleich zwischen der Abwesenheit und dem Auftreten genomischer Imbalanzen hinsichtlich der GPX1-Überexpression:**

| | **1 G.I.** | | ≥ **2 G.I.** | |
|---|---|---|---|---|
| GPX1 > 5.2 | 11/17 | 65% | 8/8 | 100% |

**Tabelle 8d: Vergleich zwischen der Abwesenheit und dem Auftreten genomischer Imbalanzen hinsichtlich der MNSOD-, TXNRD1- und GPX1-Überexpression:**

| | **1 G.I.** | | **≥ 2 G.I.** | |
|---|---|---|---|---|
| 0 | 0/17 | 0% | 0/7 | 0% |
| 1 | 2/17 | 12% | 0/7 | 0% |
| 2 | 8/17 | 53% | 2/7 | 29% |
| 3 | 8/17 | 35% | 5/7 | 71% |

### Erfindungsgemäße mRNA-Expressionsanalyse mittels Biochips

### Gesamtamplifikation der mRNA

Die Amplifikation der RNA erfolgt wie in Zohlnhöfer D, et al. Circulation 103,1396-1402, 2001 beschrieben.

### Chip-Design

Auf dem Chip sind neben verschiedenen tumorrelevanten genspezifischen Sonden auch Sonden zur Quantifizierung der MnSOD-, GPX2-, GPX3- und TXNRD-Expression integriert.

Folgende MnSOD-, GPX2-, GPX3- und TXNRD -spezifische Sonden werden verwendet:

### Herstellung der oligonukleotid-Arrays

### Herstellung der Sonden

a) Oligonukleotide (60-mere)
   Die Oligonukleotide werden in an sich bekannter Weise über eine Festphasensynthese nach der Phosphoramiditmethode hergestellt. Es werden über das 3'-OH an die Festphase gekoppelte Oligonukleotide mit DMTr-geschütztem 5'-OH und obiger Sequenz synthetisiert.
b) Chinon-Spacer-Konstrukt
   Dieses werden in an sich bekannter Weise aus Anthrachinon-2-carbonsäure, Mono-Boc-1,3-propandiamin und Hexaethylenglykol synthetisiert.
c) Chinon-Spacer-Oligonukleotid-Konstrukt
   Nach dem Aufbau obiger Sequenzen, werden abermals die DMTr-Schutzgruppe am 5'-Ende unter sauren Bedingungen mit ZnBr₂ entfernt und das freie 5'-OH mit dem 2-Cyanoethyl-N,N-diisopropyl-chlorphosphoramidit-aktivierten Chinon-Spacer-Konstrukt umgesetzt.

Die auf diese Weise synthetisierten Chinonderivate weisen daher eine Struktur der allgemeinen Formel AQ-CO-NH-(CH₂)₃-NH-(CH₂)₂(OCH₂CH₂)₅-O-PO₂-5'(SEQ ID NO:19-22)-3' auf.

Auch die übrigen krebsrelevanten genspezifischen Sonden werden in analoger Weise hergestellt.

### Kopplung der Sonden an die Array-Oberfläche

Eine wässrige Lösung (2 mM Calciumchlorid; 1 Vol.-% 1-Propanol) jedes gewünschten Chinonderivates (10 µM) wird mit einem Piezodispensor (1-Kanal; Genesis NPS 100/4 mit Active Tip M, TECAN AG, Hombrechtikon, CH) in einem Raster von 400 µm auf Polycycloolefin (Zeonex 480R; Zeon) aufgetragen. Die Tropfengröße beträgt etwa 0,5 nL. Nach dem Eintrocknen der Spots wird der Träger 1 min mit UV-Licht bestrahlt. Anschließend wird der Träger gewaschen und an der Luft bei Raumtemperatur getrocknet. Die Durchmesser der resultierenden Spots betragen etwa 120 bis 180 µm.

### Hybridisierung

Die aus der RNA-Amplifikation erhaltenen Ansätze werden in geeigneter Weise mit 2xSSPE-Puffer verdünnt und auf den Array pipettiert. Es wird 16 Stunden bei 60 °C inkubiert. Nach zwei Waschschritten mit 1xSSPE-Puffer bei 60°C wird Cy5-Streptavidin (Amersham Pharmacia Biotec) hinzugegeben und 15 min bei Raumtemperatur inkubiert.

### Auswertung des Arrays

Mit Hilfe eines CCD-Kamerascanners wird die Fluoreszenz bestimmt.

### Beispiel 3: Tumorpatienten

Figur 1 zeigt eine CCD-Kontaktbelichtungsaufnahme der vom Array emittierten Fluoreszenzstrahlung nach Hybridisierung mit cDNA-Einzelstrangfragmenten, die in der oben beschriebenen Weise mittels mRNA-Gesamtamplifikation aus der Tumorzellfraktion C bzw. der Vergleichzellfraktion A' generiert wurden.

Die Überexpression von MNSOD und GPX2 in der Tumorzellfraktion ist deutlich zu erkennen. Die Messung unterstreicht die gegenüber anderen tumorzellnachweisenden Genen erhöhte Sensitivität und Spezifität des erfindungsgemäßen Nachweisverfahrens.

## Patentansprüche

1. Verfahren zum Untersuchen einer Körperflüssigkeit auf Krebszellen, wobei man an wenigstens einer zellhaltigen Fraktion der Körperflüssigkeit die Expression von wenigstens 2 Genen bestimmt, die ausgewählt sind unter
i) Mangan-Superoxiddismutase-Genen;
ii) Thioredoxinreduktase-Genen; und
iii) Glutathionperoxidase-Genen.

2. Verfahren nach Anspruch 1, wobei die Krebszellen disseminierte Krebszellen sind.

3. Verfahren nach Anspruch 1 oder 2, **dadurch gekennzeichnet, dass** man die Expression wenigstens eines Mangan-Superoxiddismutase-Gens, wenigstens eines Thioredoxinreduktase-Gens und wenigstens eines Glutathionperoxidase-Gens bestimmt.

4. Verfahren nach einem der vorhergehenden Ansprüche, **dadurch gekennzeichnet, dass** die Körperflüssigkeit ausgewählt ist unter Blut, Knochenmark, Lymphe, Sputum, Lavagen, Punktaten, Ascites, Schleimhautabstrichen, Exsudaten, Urin und Stuhl.

5. Verfahren nach einem der vorhergehenden Ansprüche, **dadurch gekennzeichnet, dass** man die zellhaltige Fraktion aus der Körperflüssigkeit unter Anreicherung von Krebszellen gewinnt.

6. Verfahren nach einem der vorhergehenden Ansprüche, **dadurch gekennzeichnet, dass** man
- die zellhaltige Fraktion aus der Körperflüssigkeit unter Anreicherung von Krebszellen gewinnt und die Expression der Gene in der zellhaltigen Fraktion bestimmt,
- eine weitere zellhaltige Fraktion der Körperflüssigkeit oder einer vergleichbaren biologischen Probe bereitstellt und die Expression der Gene in der weiteren zellhaltigen Fraktion bestimmt, und
- für jedes Gen dessen Expression in der zellhaltigen Fraktion mit dessen Expression in der weiteren zellhaltigen Fraktion vergleicht.

7. Verfahren nach Anspruch 6, **dadurch gekennzeichnet, dass** die vergleichbare biologische Probe aus dem Individuum stammt, dessen Körperflüssigkeit auf Krebszellen untersucht wird.

8. Verfahren nach einem der Ansprüche 6 oder 7, **dadurch gekennzeichnet, dass** man feststellt, ob die Expression der Gene in der zellhaltigen Fraktion im Vergleich zur Expression der Gene in der weiteren zellhaltigen Fraktion erhöht ist.

9. Verwendung eines Verfahrens nach einem der Ansprüche 1 bis 8 zur Identifizierung von disseminierten Krebszellen in einem Individuum zur Tumorfrüherkennung oder zur Abschätzung des Risikos, dass das Individuum eine Metastase oder ein Rezidiv entwickelt.

10. Analysekit, enthaltend
i) sequenzspezifische Primer und/oder Sonden zur Bestimmung der Expression wenigstens eines Mangan-Superoxiddismutase-Gens;
ii) sequenzspezifische Primer und/oder Sonden zur Bestimmung der Expression wenigstens eines Thioredoxinreduktase-Gens; und
iii) sequenzspezifische Primer und/oder Sonden zur Bestimmung der Expression wenigstens eines Glutathionperoxidase-Gens,
und gegebenenfalls weitere übliche Mittel zur Durchführung eines Verfahrens nach einem der Ansprüche 1 bis 8.

## Claims

1. A method for investigating a body fluid for cancer cells, where the expression of at least 2 genes which are selected from
i) manganese superoxide dismutase genes;
ii) thioredoxin reductase genes; and
iii) glutathione peroxidase genes
is determined on at least one cell-containing fraction of the body fluid.

2. The method as claimed in claim 1, **characterized in that** the cancer cells are disseminated cancer cells.

3. The method as claimed in claim 1 or 2, **characterized in that** the expression of at least one manganese superoxide dismutase gene, of at least one thioredoxin reductase gene and of at least one glutathione peroxidase gene is determined.

4. The method as claimed in any one of the preceding claims, **characterized in that** the body fluid is selected from blood, bone marrow, lymph, sputum, lavages, puncture fluids, ascites, mucosal smears, exudates, urine and stool.

5. The method as claimed in any of the preceding claims, **characterized in that** the cell-containing fraction is obtained from the body fluid with enrichment of cancer cells.

6. The method as claimed in any of the preceding claims, **characterized in that**
- the cell-containing fraction is obtained from the body fluid with enrichment of cancer cells, and the expression of the genes in the cell-containing fraction is determined,
- a further cell-containing fraction of the body fluid or of a comparable biological sample is provided, and the expression of the genes in the further cell-containing fraction is determined, and
- the expression for each gene in the cell-containing fraction is compared with its expression in the further cell-containing fraction.

7. The method as claimed in claim 6, **characterized in that** the comparable biological sample is derived from the individual whose body fluid is investigated for cancer cells.

8. The method as claimed in either of claims 6 or 7, **characterized in that** it is determined whether expression of the genes in the cell-containing fraction is elevated by comparison with the expression of the genes in the further cell-containing fraction.

9. The use of a method as claimed in any of claims 1 to 8 for identifying disseminated cancer cells in an individual, in particular for early diagnosis of tumors, and for estimating the risk for the individual to develop a metastasis or a recurrence.

10. An analysis kit comprising
i) sequence-specific primers and/or probes for determining the expression of at least one manganese superoxide dismutase gene;
ii) sequence-specific primers and/or probes for determining the expression of at least one thioredoxin reductase gene; and
iii) sequence-specific primers and/or probes for determining the expression of at least one glutathione peroxidase gene,
and optionally further usual means for carrying out the method as claimed in any of claims 1 to 8.

## Revendications

1. Procédé d'analyse d'un fluide corporel en vue d'y rechercher des cellules cancéreuses, dans lequel on évalue, dans au moins une fraction du liquide corporel qui contient des cellules, l'expression d'au moins deux gènes choisis parmi les suivants :
i) les gènes de manganèse-superoxyde dismutase ;
ii) les gènes de thioredoxine réductase ;
iii) et les gènes de glutathion peroxydase.

2. Procédé conforme à la revendication 1, dans lequel les cellules cancéreuses sont des cellules cancéreuses disséminées.

3. Procédé conforme à la revendication 1 ou 2, **caractérisé en ce que** l'on évalue l'expression d'au moins un gène de manganèse-superoxyde dismutase, d'au moins un gène de thioredoxine réductase et d'au moins un gène de glutathion peroxydase.

4. Procédé conforme à l'une des revendications précédentes, **caractérisé en ce que** le fluide corporel est choisi parmi du sang, de la moelle osseuse, de la lymphe, des crachats, des liquides de lavage, des liquides ponctionnés, des liquides d'ascite, des frottis de muqueuses, des exsudats, de l'urine et des selles.

5. Procédé conforme à l'une des revendications précédentes, **caractérisé en ce que** l'on obtient la fraction contenant des cellules, à partir du fluide corporel, par concentration des cellules cancéreuses.

6. Procédé conforme à l'une des revendications précédentes, **caractérisé en ce que** :
- l'on obtient une première fraction contenant des cellules, à partir du fluide corporel, par concentration des cellules cancéreuses, et l'on évalue l'expression des gènes dans cette fraction contenant des cellules ;
- on prépare une autre fraction du liquide corporel, qui contient des cellules, ou un autre échantillon biologique comparable, et l'on évalue l'expression des gènes dans cette autre fraction contenant des cellules ;
- et l'on compare, pour chaque gène, son expression dans la première fraction contenant des cellules avec son expression dans l'autre fraction contenant des cellules.

7. Procédé conforme à la revendication 6, **caractérisé en ce que** l'échantillon biologique comparable provient de l'individu dont on analyse un liquide corporel pour y rechercher des cellules cancéreuses.

8. Procédé conforme à la revendication 6 ou 7, **caractérisé en ce que** l'on vérifie si l'expression des gènes dans la première fraction contenant des cellules est plus forte que l'expression des gènes dans l'autre fraction contenant des cellules.

9. Emploi d'un procédé conforme à l'une des revendications 1 à 8 en vue d'identifier chez un individu des cellules cancéreuses disséminées, afin de détecter précocement une tumeur chez cet individu ou d'estimer le risque que court cet individu de développer une métastase ou une récidive.

10. Trousse d'analyse qui contient :
i) des amorces et/ou des sondes spécifiques, servant à déterminer l'expression d'au moins un gène de manganèse-superoxyde dismutase ;
ii) des amorces et/ou des sondes spécifiques, servant à déterminer l'expression d'au moins un gène de thioredoxine réductase ;
iii) et des amorces et/ou des sondes spécifiques, servant à déterminer l'expression d'au moins un gène de glutathion peroxydase ;
et le cas échéant, d'autres agents ou moyens habituellement employés pour mettre en oeuvre un procédé conforme à l'une des revendications 1 à 8.


### SEQUENCE LISTING

<110> Giesing, Michael
<120> Verfahren zum Untersuchen von Körperflüssigkeiten auf Krebszellen, dessen Verwendung, entsprechende Analysekits und Verwendung bestimmter Wirkstoffe zur Behandlung von Krebs
<130> M-43161-EP
<150> DE 102 38 046.5
   <151> 2002-08-20
<160> 22
<170> PatentIn version 3.1
<210> 1
   <211> 22
   <212> DNA
   <213> Artificial Sequence
<220>
   <223> forward primer (MNSOD)
<400> 1
   gtcaccgagg agaagtacca gg 22
<210> 2
   <211> 20
   <212> DNA
   <213> Artificial Sequence
<220>
   <223> reverse primer (MNSOD)
<400> 2
   gggctgaggt ttgtccagaa 20
<210> 3
   <211> 27
   <212> DNA
   <213> Artificial Sequence
<220>
   <223> probe (MNSOD)
<400> 3
   cgttggccaa gggagatgtt acagccc 27
<210> 4
   <211> 23
   <212> DNA
   <213> Artificial Sequence
<220>
   <223> forward primer (TXNRD1)
<400> 4
   ggagggcaga cttcaaaagc tac 23
<210> 5
   <211> 22
   <212> DNA
   <213> Artificial Sequence
<220>
   <223> reverse primer (TXNRD1)
<400> 5
   acaaagtcca ggaccatcac ct 22
<210> 6
   <211> 26
   <212> DNA
   <213> Artificial Sequence
<220>
   <223> probe (TXNRD1)
<400> 6
   ttgggctgcc tccttagcag ctgcca 26
<210> 7
   <211> 17
   <212> DNA
   <213> Artificial Sequence
<220>
   <223> forward primer (GPX1)
<400> 7
   ctcggcttcc cgtgcaa 17
<210> 8
   <211> 19
   <212> DNA
   <213> Artificial Sequence
<220>
   <223> reverse primer (GPX1)
<400> 8
   tgaagttggg ctcgaaccc 19
<210> 9
   <211> 28
   <212> DNA
   <213> Artificial Sequence
<220>
   <223> probe (GPX1)
<400> 9
   agtttgggca tcaggagaac gccaagaa 28
<210> 10
   <211> 19
   <212> DNA
   <213> Artificial Sequence
<220>
   <223> forward primer (GAPDH)
<400> 10
   tgctgatgcc cccatgttc 19
<210> 11
   <211> 20
   <212> DNA
   <213> Artificial Sequence
<220>
   <223> reverse primer (GAPDH)
<400> 11
   ggcagtgatg gcatggactg 20
<210> 12
   <211> 27
   <212> DNA
   <213> Artificial Sequence
<220>
   <223> probe (GAPDH)
<400> 12
   tcaagatcat cagcaatgcc tcctgca 27
<210> 13
   <211> 222
   <212> PRT
   <213> Homo sapiens
<400> 13
<210> 14
   <211> 976
   <212> DNA
   <213> Homo sapiens
<400> 14
   gcgggcggcg caggagcggc actcgtggct gtggtggctt cggcagcggc ttcagcagat      60
   cggcggcatc agcggtagca ccagcactag cagcatgttg agccgggcag tgtgcggcac     120
   cagcaggcag ctggctccgg ctttggggta tctgggctcc aggcagaagc acagcctccc     180
   cgacctgccc tacgactacg gcgccctgga acctcacatc aacgcgcaga tcatgcagct     240
   gcaccacagc aagcaccacg cggcctacgt gaacaacctg aacgtcaccg aggagaagta     300
   ccaggaggcg ttggccaagg gagatgttac agcccagaca gctcttcagc ctgcactgaa     360
   gttcaatggt ggtggtcata tcaatcatag cattttctgg acaaacctca gccctaacgg     420
   tggtggagaa cccaaagggg agttgctgga agccatcaaa cgtgactttg gttcctttga     480
   caagtttaag gagaagctga cggctgcatc tgttggtgtc caaggctcag gttggggttg     540
   gcttggtttc aataaggaac ggggacactt acaaattgct gcttgtccaa atcaggatcc     600
   actgcaagga acaacaggcc ttattccact gctggggatt gatgtgtggg agcacgctta     660
   ctaccttcag tataaaaatg tcaggcctga ttatctaaaa gctatttgga atgtaatcaa     720
   ctgggagaat gtaactgaaa gatacatggc ttgcaaaaag taaaccacga tcgttatgct     780
   gagtatgtta agctctttat gactgttttt gtagtggtat agagtactgc agaatacagt     840
   aagctgctct attgtagcat ttcttgatgt tgcttagtca cttatttcat aaacaactta     900
   atgttctgaa taatttctta ctaaacattt tgttattggg caagtgattg aaaatagtaa     960
   atgctttgtg tgattg                                                     976
<210> 15
   <211> 497
   <212> PRT
   <213> Homo sapiens
<400> 15
<210> 16
   <211> 1314
   <212> DNA
   <213> Homo sapiens
<400> 16
   gaattcgggt ggagtcctga aggagggcct gatgtcttca tcattctcaa attcttgtaa      60
   gctctgcgtc gggtgaaacc agacaaagcc gcgagcccag ggatgggagc acgcggggga     120
   cggcctgccg gcggggacga cagcattgcg cctgggtgca gcagtgtgcg tctcggggaa     180
   gggaagatat tttaaggcgt gtctgagcag acggggaggc ttttccaaac ccaggcagct     240
   tcgtggcgtg tgcggtttcg acccggtcac acaaagcttc agcatgtcat gtgaggacgg     300
   tcgggccctg aaaggaacgc tctcggaatt ggccgcggaa accgatctgc ccgttgtgtt     360
   tgtgaaacag agaaagatag gcggccatgg tccaaccttg aaggcttatc aggagggcag     420
   acttcaaaag ctactaaaaa tgaacggccc tgaagatctt cccaagtcct atgactatga     480
   ccttatcatc attggaggtg gctcaggagg tctggcagct gctaaggagg cagcccaata     540
   tggcaagaag gtgatggtcc tggactttgt cactcccacc cctcttggaa ctagatgggg     600
   tcttggagga acatgtgtga atgtgggttg catacctaaa aaactgatgc atcaagcagc     660
   tttgttagga caagccctgc aagactctcg aaattatgga tggaaagtcg aggagacagt     720
   taagcatgat tgggacagaa tgatagaagc tgtacagaat cacattggct ctttgaattg     780
   gggctaccga gtagctctgc gggagaaaaa agtcgtctat gagaatgctt atgggcaatt     840
   tattggtcct cacaggatta aggcaacaaa taataaaggc aaagaaaaaa tttattcagc     900
   agagagtttt ctcattgcca ctggtgaaag accacgttac ttgggcatcc ctggtgacaa     960
   agaatactgc atcagcagtg atgatctttt ctccttgcct tactgcccgg gtaagaccct    1020
   ggttgttgga gcatcctatg tcgctttgga gtgcgctgga tttcttgctg gtattggttt    1080
   aggcgtcact gttatggtta ggtccattct tcttagagga tttgaccagg acatggccaa    1140
   caaaattggt gaacacatgg aagaacatgg catcaagttt ataagacagt tcgtaccaat    1200
   taaagttgaa caaattgaag cagggacacc aggccgactc agagtagtag ctcagtccac    1260
   caatagtgag gaaatcattg aaggagaata taatacggtg atgctggcaa tagg          1314
<210> 17
   <211> 201
   <212> PRT
   <213> Homo sapiens
<400> 17
<210> 18
   <211> 856
   <212> DNA
   <213> Homo sapiens
<400> 18
   cttgttcggg gcgctccgct ggcttcttgg acaattgcgc catgtgtgct gctcggctag 60
   cggcggcggc ggcccagtcg gtgtatgcct tctcggcgcg cccgttggcc ggcggggagc 120
   ctgtgagcct gggctccctg cggggcaagg tactacttat cgagaatgtg gcgtccctct 180
   gaggcaccac ggtccgggac tacacccaga tgaacgagct gcagcggcgc ctcggacccc 240
   ggggcctggt ggtgctcggc ttcccgtgca accagtttgg gcatcaggag aacgccaaga 300
   acgaagagat tctgaattcc ctcaagtacg tccggcctgg tggtgggttc gagcccaact 360
   tcatgctctt cgagaagtgc gaggtgaacg gtgcgggggc gcaccctctc ttcgccttcc 420
   tgcgggaggc cctgccagct cccagcgacg acgccaccgc gcttatgacc gaccccaagc 480
   tcatcacctg gtctccggtg tgtcgcaacg atgttgcctg gaactttgag aagttcctgg 540
   tgggccctga cggtgtgccc ctacgcaggt acagccgccg cttccagacc attgacatcg 600
   agcctgacat cgaagccctg ctgtctcaag ggcccagctg tgcctagggc gcccctccta 660
   ccccggctgc ttggcagttg cagtgctgct gtctcggggg ggttttcatc tatgagggtg 720
   tttcctctaa acctacgagg gaggaacacc ttgatcttac agaaaatacc acctcgagat 780
   gggtgctggt cctgttgatc ccagtctctg ccagaccaag gcgagtttcc ccactaataa 840
   agtgccgggt gtcagc 856
<210> 19
   <211> 60
   <212> DNA
   <213> Artificial Sequence
<220>
   <223> probe (NMSOD)
<400> 19
   gaacaacagg ccttattcca ctgctgggga ttgatgtgtg ggagcacgct tactaccttc 60
<210> 20
   <211> 60
   <212> DNA
   <213> Artificial Sequence
<220>
   <223> probe (TXNRD1)
<400> 20
   cgtgttgtgg gctttcacgt actgggtcca aatgctggag aagttacaca aggctttgca 60
<210> 21
   <211> 60
   <212> DNA
   <213> Artificial Sequence
<220>
   <223> probe (GPX2)
<400> 21
   tacagccgca ccttcccaac catcaacatt gagcctgaca tcaagcgcct ccttaaagtt 60
<210> 22
   <211> 60
   <212> DNA
   <213> Artificial Sequence
<220>
   <223> probe (GPX3)
<400> 22
   ctcttctggg aacccatgaa ggttcacgac atccgctgga actttgagaa gttcctggtg 60